# EUROPEAN PATENT APPLICATION

(11) **EP 1 589 111 A1**
(43) Date of publication of application: **26.10.2005**
(21) Application number: 03767378.7
(22) Date of filing: 19.12.2003
(51) Int. Cl.: C12N 15/82, C07K 14/10

(54) **RECOMBINANT HEPATITIS A VIRUS ANTIGENS OBTAINED IN PLANT CELLS**

(30) Priority: 31.01.2003 CU 3103
(71) Applicant: CENTRO DE INGENIERIA GENETICA Y BIOTECNOLOGIA (CIGB), Ciudad de la Habana 10600 (CU)
(72) Inventor: LOPEZ QUESADA, Alina, 12100 Ciudad Habana (CU); GONZALEZ BADILLO, Beatriz, 12100 Ciudad Habana (CU); SELMAN-HOUSEIN SOSA, Guillermo, 12100 Ciudad Habana (CU); HERNANDEZ VELASQUEZ, Abel, 19290 Ciudad Habana (CU); RIOS BACALLAO, Javier, 19290 Ciudad Habana (CU); ROSABAL AYON, Yamilka, 11300 Ciudad Habana (CU); PEREZ MARTINEZ, Marlen, 10400 Ciudad Habana (CU); RODRIGUEZ LAY, Licel, 17100 Ciudad Habana (CU); GARCIA GONZALEZ, Rolando, Comunidad Cientifica, 70100 Camagüey (CU)
(74) Representative: Winckels, Johannes Hubertus F.
(86) International application number: PCT/CU2003/000017
(87) International publication number: WO 2004/067747

(57) **Abstract**

Generation of genetic constructions based on modified fragments of the hepatitis A virus (HAV) genome, using the strain M2, isolated in Cuba. Nucleotides sequences of these fragments fused to the appropriated localization and regulation signals, are expressed in transgenic plant. The obtained recombinant antigens of the HAV are composed of pentamers and /or empty envelope, able to develop immune response.

SEQUENCE LISTING

<110> CENTER FOR GENETIC ENGINEERING AND BIOTECHNOLOGY

<120> RECOMBINANT HEPATITIS A VIRUS ANTIGENS PRODUCED IN PLANT CELLS.

<130> ORF.

<140>
<141>

<160> 24

<170> PatentIn Ver. 2.1

<210> 1
<211> 25
<212> ADN
<213> Chimeric Sequence

<220>
<221> primer_bind
<222> (1)..(25)
<223> Sequence # 1.
      Sequence of the oligonocleotide # 1 used for the amplification of ORF
coding sequence by RT-PCR.

<400> 1
cttaatctag aatgaatatg tccaa                                          25

<210> 2
<211> 22
<212> ADN
<213> Chimeric Sequence

<220>
<221> primer_bind
<222> (1)..(22)
<223> Sequence # 2.
      Sequence of the oligonocleotide # 2 used for the amplification of ORF
coding sequence by RT-PCR.
<400> 2
gaaagaaata aaggtacctc ag                                             22

<210> 3
<211> 6685
<212> ADN
<213> Hepatitis A virus

<220>
<221> gene
<222> Complement((1)..(6685))
<223> Sequence # 3.
      Nucleotide sequence coding for the HAV open reading frame (ORF) of
the Cuban M2 strain.

<400> 3
atgaatatgt ccaaacaagg aattttccag actgttggga gtggccttga ccacatcctg 60
tccttggcag atattgagga agagcaaatg attcagtccg ttgataggac tgcagtgact 120
ggagcttctt atttcacttc tgtggaccaa tcttcagttc atactgctga ggttggctca 180
caccaaattg aacctttgaa aacctctgtt gataaacctg gttctaagaa aactcagggg 240
gagaagtttt tcttgattca ttctgctgat tggctcacta cacatgctct ctttcatgaa 300
gttgcaaaat tggatgtggt gaaactgctg tacaatgagc agtttgccgt ccaaggtttg 360
ttgagatacc atacttatgc aagatttggc attgagattc aagttcagat aaatcccaca 420
ccctttcagc aaggaggact aatctgtgcc atggttcctg gtgaccaaag ttatggttca 480
atagcatcct tgactgttta tcctcatggt ctgttaaatt gcaatatcaa caatgtagtt 540
agaataaagg ttccatttat ttatactaga ggtgcttatc attttaaaga tccacagtac 600
ccagtttggg aattgacaat cagagtttgg tcagagttga atattggaac aggaacctca 660
gcttatactt cactcaatgt tttagctagg tttacagatt tggagttgca tggattaact 720
cctctttcta cacagatgat gagaaatgaa tttagagtta gtactactga aaatgttgta 780
aatttgtcaa attatgaaga tgcaaggcca aaaatgtctt ttgctttgga tcaggaagat 840
tggaagtctg atccttccca aggtggtgga attaaaatta ctcatttcac tacctggaca 900
tccattccaa ccttagctgc tcagtttcca ttcaatgctt cagattcagt tgggcaacaa 960
attaaagtta taccagtgga cccatacttt ttccagatga caaacactaa tcctgatcaa 1020
aaatgtataa cagccttggc ctctatttgt cagatgttct gcttttggag gggagatctt 1080
gttttcgatt tccaggtttt tccaaccaaa tatcattcag gtaggctgtt gttttgtttt 1140
gttcctggga atgagttaat agatgttact ggaattacat taaaacaggc aactactgct 1200
ccttgtgcag tgatggacat tacaggagtg cagtcaacct tgagatttcg tgttccttgg 1260
atttctgata caccctatcg agtgaatagg tacacgaagt cagcacatca aaaaggtgag 1320
tatactgcca ttgggaagct tattgtgtat tgttataata gattgacttc tccttctaat 1380
gttgcttctc atgttagagt taatgtttat ctttcagcaa ttaatttgga atgttttgct 1440
cctctttacc atgctatgga tgttaccaca caggttggag atgattcagg aggtttctca 1500
acaacagttt ctacagagca gaatgttcct gatccccaag ttggcataac aaccatgagg 1560
gatttaaaag ggaaagccaa taggggaaag atggatgtat caggagtgca ggtacctgtg 1620
ggagctatta caacaattga ggatccagtt ttagcaaaga aagtacctga gacatttcct 1680
gaattgaagc ctggagaatc cagacataca tcagatcaca tgtctattta taaattcatg 1740
ggaaggtctc atttcttgtg tacttttact tttaattcaa acaataaaga gtacacattt 1800
ccaataactc tgtcttcgac ttctaatcct cctcatggtt taccatcaac attaaggtgg 1860
ttctttaatt tgtttcagtt gtatagagga ccattggatt tgacaattat aatcacagga 1920
gccactgatg tggatggtat ggcctggttt actccagtgg gccttgctgt cgacacccct 1980
tgggtggaaa agaagtcagc tttgtctatt gattataaaa ctgcccttgg agctgttaga 2040
tttaatacaa gaagaacagg gaacattcag attagattgc catggtattc ttatttgtat 2100
gccgtgtctg gagcactgga tggcttggga gataagacag attctacatt tggattggtt 2160
tctattcaga ttgcaaatta caatcattct gatgaatatt tgtcctttag ttgttatttg 2220
tctgtcacag agcaatcaga gttctatttc cctagagctc cattaaattc aaatgctatg 2280
ttgtccactg agtccatgat gagtagaatt gcagctggag acttggagtc atcagtggat 2340
gatcccagat cagaggagga cagaagattt gagagtcata tagaatgtag gaaaccatat 2400
aaagaattga gactggaggt tgggaaacaa agaatcaaat atgctcagga agagttatca 2460
aatgaagtgc ttccacctcc taggaaaatg aaggggttat tttcacaagc taaaatttct 2520
ctttttttata cagaggacca tgaaataatg aaattttctt ggagaggagt gactgctaat 2580
actagggctt tgagaagatt tggattctct ctggctgctg gtagaagtgt gtggactctt 2640
gaaatggatg ctggagttct tactggaaga ttgatcagat tgaatgatga gaaatggaca 2700
gaaatgaagg atgataagat tgtttcatta attgaaaagt tcacaagcaa taaacattgg 2760
tctaaagtga attttccaca tggaatgttg gatcttgagg aaattgctgc caactctaaa 2820
gattttccaa atatgtctga gacagatttg tgtttcctgt tgcattggct aaatccaaag 2880
aaaattaatt tagcagatag aatgcttgga ttgtctggag tgcaggaaat taaagaacag 2940
ggtgttggac tgatagcaga gtgtagaact ttcttggatt ctattgctgg gactttgaaa 3000
tctatgattt ttgggtttca ttattctgtg actgttgaaa ttataaatat tgtgctttgt 3060
tttattaaga gtggaatcct gctttatgtc atacaacaat tgaaccaaga tgaacactct 3120
cacataattg gtttgttgag agttatgaat tatgcagata ttggctgttc agtcatttca 3180
tgtggtaaag ttttttccaa aatgttagaa acagttttta attggcaaat ggactctaga 3240
atgatggagc tgaggactca gagcttctcc aattggttaa gagatatttg ttcgggaatt 3300
actattttta aaagttttaa ggatgccata tattggttat gtacaaaatt gaaggatttt 3360
tatgaagtaa attatggcaa gaaaaaggat gttcttaata ttctcaaaga taaccagcaa 3420
aaaatagaaa aagccattga agaagcagac aatttttgca ttttgcaaat tcaagatgtg 3480
gagaaatttg atcagtatca gaaaggggtt gatttaatac aaaagctgag aactgtccat 3540

tcaatggctc aagttgaccc cagtttgggg gttcatttgt cacctctcag agattgcata 3600
gcaagagtcc atcaaaagct caagaatctt ggatctataa atcaggccat ggtaacaaga 3660
tgtgagccag ttgtttgcta tttgtatggc aaaagagggg gagggaaaag cttgacttca 3720
attgcattgg caaccaaaat ttgtaaacac tatggtgttg aacctgagaa aaatatttac 3780
accaaacctg tggcctcaga ttattgggat ggatatagtg gacaattagt ttgtattatt 3840
gatgatatcg gccaaaacac aacagatgaa gattggtcag atttttgtca attagtgtca 3900
ggatgcccaa tgagattgaa tatggcttct cttgaggaga agggcagaca tttttcctct 3960
ccttttataa tagcatcttc aaattggtca aatccaagtc caaaaacagt ttatgttaaa 4020
gaagcaattg atcgtaggct tcattttaag gttgaagtta aacctgcttc attttttaaa 4080
aatcctcaca atgatatgtt aaatgttaat ttggctaaaa caaatgatgc aattaaagac 4140
atgtcttgtg ttgatttgat aatggatgga cacaatattt cattgatgga tttacttagt 4200
tccttagtga tgacaggtga aattaggaaa cagaatatga gtgaattcat ggagttgtgg 4260
tctcagggaa tttcagatga tgacaatgat agtgcagtag ctgagttttt ccggtctttt 4320
ccatctggtg aaccatcaaa ttccaagtta tctagttttt tccaagctgt cactaatcac 4380
aagtgggttg ctgtgggagc tgcagttggt attcttggat tgctagtggg aggatggttt 4440
gtgtataagc atttttcccg caaagaggaa gaaccaattc cagctgaagg ggtttatcat 4500
ggagtgacta agcccaaaca agtgattaaa ttggatgcag atccagtaga gtctcagtca 4560
actctagaaa tagcaggatt agttaggaaa aatttggttc agtttggagt tggtgagaaa 4620
aatggatgtg tgagatgggt catgaatgcc ttaggagtga aggatgattg gttgttagta 4680
ccttctcatg cttataaatt tgaaaaggat tatgaaatga tggagtttta tttcaataga 4740
ggtggaactt actattcaat ttcagctggt aatgttgtta ttcaatcttt agatgtggga 4800
ttccaagatg ttgttctaat gaaggttcct acaattccca agtttagaga tattactcaa 4860
cattttatta agaaaggaga tgtgcctaga gccttgaatc gcttggcaac attagtgaca 4920
accgttaatg gaactcctat gttaatttct gagggacctt taaaaatgga agaaaaagcc 4980
acttatgttc ataagaagaa tgatggtact acggttgatt tgactgtaga tcaggcatgg 5040
agaggaaaag gtgaaggtct tcctggaatg tgtggtgggg ccctagtgtc atcaaatcag 5100
tccatacaaa atgcaatttt gggtattcat gttgctggag gaaattcaat tcttgtggca 5160
aagttgatta ctcaagaaat gtttcaaaac attgataaga aaattgaaag tcagagaata 5220
atgaaagtgg aatttactca atgttcaatg aatgtagtct ccaaaacgct ttttagaaag 5280
agtcccattc atcaccacat tgataaaacc atgattaatt ttcctgcagc tatgcctttc 5340
tctaaagctg aaattgatcc aatggctatg atgttgtcta aatattcatt acctattgtg 5400
gaagaaccag aggattacaa agaagcttca gttttttatc aaaataaaat agtaggcaag 5460
actcagctag ttgatgactt tctagatctt gatatggcca ttacaggggc tccaggcatt 5520
gatgctatta atatggattc atctcctggg tttccttatg ttcaagaaaa attgactaaa 5580
agagatttga tttggttgga tgaaaatggt ttactgttag gagttcaccc aagattggcc 5640
cagagaatct tatttaatac tgtcatgatg gaaaattgtt ctgacttaga tgttgttttt 5700
acaacttgtc caaaagatga attgagacca ttagagaaag ttttggaatc aaaaacaaga 5760
gctattgatg cttgcccttt ggattataca attttatgtc gaatgtattg gggtccagct 5820
attagttatt ttcatttgaa tccagggttt cacacaggtg ttgctattgg catagatcct 5880
gatagacagt gggatgaatt atttaaaaca atgataagat ttggagatgt tggtcttgat 5940
ttagattttt ctgcttttga tgccagtctt agtccattta tgattaggga agcaggtaga 6000
atcatgagtg aattatctgg aacaccatct cattttggaa cagctcttat caatactatc 6060
atttattcta aacatctgct gtacaattgt tgttatcacg tctgtggttc aatgccttct 6120
gggtctcctt gtacagcttt gttgaattca attattaata atattaattt gtattatgtg 6180
ttttctaaaa tatttggaaa gtctccagtt ttcttttgtc aagctttgag gatcctttgt 6240
tatggagatg atgttttgat agttttttcc agagatgttc aaattgataa tcttgacttg 6300
attggacaga aaattgtgga tgagttcaaa aaacttggca tgacagccac ttcagctgac 6360
aaaaatgtgc ctcaactgaa gccagtttca gaattgactt ttcttaaaag atcttttaat 6420
ttggtggagg acagaatcag acctgcaatt tcagaaaaga caatttggtc tttgatagct 6480
tggcagagaa gtaacgctga gtttgagcag aatttagaaa atgctcagtg gtttgctttc 6540
atgcatggct atgagttcta tcagaaattc tattattttg ttcagtcctg tttggagaaa 6600
gagatgatag aatatagact taaatcttat gattggtgga gaatgagatt ttatgaccag 6660
tgtttcattt gtgacctttc atgat 6685

<210> 4
<211> 40
<212> ADN
<213> Chimeric Sequence

<220>
<221> primer_bind

<222> (1)..(40)
<223> Sequence # 4.
      Sequence of the oligonocleotide # 5 used for the
amplification of P1-2A coding sequence by PCR.

<400> 4
ttgaattcag cttgtgaaaa taaccccttc attttcctag                    40

<210> 5
<211> 28
<212> ADN
<213> Chimeric Sequence

<220>
<221> primer_bind
<222> (1)..(28)
<223> Sequence # 5.

Sequence of the oligonocleotide # 5 used for the
amplification of P1-2A coding sequence by PCR.

<400> 5
cgcccgggtc tagaatgaat atgtccaa                                28

<210> 6
<211> 2523
<212> ADN
<213> Hepatitis A virus

<220>
<221> gene
<222> Complement((1)..(2523))
<223> Sequence # 6.
      Nucleotide sequence coding for the structural
P1-2A HAV proteins of the M2 strain.

<400> 6
atgaatatgt ccaaacaagg aattttccag actgttggga gtggccttga ccacatcctg 60
tccttggcag atattgagga agagcaaatg attcagtccg ttgataggac tgcagtgact 120
ggagcttctt atttcacttc tgtggaccaa tcttcagttc atactgctga ggttggctca 180
caccaaattg aacctttgaa aacctctgtt gataaacctg gttctaagaa aactcagggg 240
gagaagtttt tcttgattca ttctgctgat tggctcacta cacatgctct ctttcatgaa 300
gttgcaaaat tggatgtggt gaaactgctg tacaatgagc agtttgccgt ccaaggtttg 360
ttgagatacc atacttatgc aagatttggc attgagattc aagttcagat aaatcccaca 420
ccctttcagc aaggaggact aatctgtgcc atggttcctg gtgaccaaag ttatggttca 480
atagcatcct tgactgttta tcctcatggt ctgttaaatt gcaatatcaa caatgtagtt 540
agaataaagg ttccatttat ttatactaga ggtgcttatc attttaaaga tccacagtac 600
ccagtttggg aattgacaat cagagtttgg tcagagttga atattggaac aggaacctca 660
gcttatactt cactcaatgt tttagctagg tttacagatt tggagttgca tggattaact 720
cctctttcta cacagatgat gagaaatgaa tttagagtta gtactactga aaatgttgta 780
aatttgtcaa attatgaaga tgcaagggca aaaatgtctt ttgctttgga tcaggaagat 840
tggaagtctg atccttccca aggtggtgga attaaaatta ctcatttcac tacctggaca 900
tccattccaa ccttagctgc tcagtttcca ttcaatgctt cagattcagt tgggcaacaa 960
attaaagtta taccagtgga cccatacttt ttccagatga caaacactaa tcctgatcaa 1020
aaatgtataa cagccttggc ctctatttgt cagatgttct gcttttggag gggagatctt 1080
gttttcgatt tccaggtttt tccaaccaaa tatcattcag gtaggctgtt gttttgtttt 1140
gttcctggga atgagttaat agatgttact ggaattacat taaaacaggc aactactgct 1200
ccttgtgcag tgatggacat tacaggagtg cagtcaacct tgagatttcg tgttccttgg 1260

atttctgata caccctatcg agtgaatagg tacacgaagt cagcacatca aaaaggtgag 1320
tatactgcca ttgggaagct tattgtgtat tgttataata gattgacttc tccttctaat 1380
gttgcttctc atgttagagt taatgtttat ctttcagcaa ttaatttgga atgttttgct 1440
cctctttacc atgctatgga tgttaccaca caggttggag atgattcagg aggtttctca 1500
acaacagttt ctacagagca gaatgttcct gatccccaag ttggcataac aaccatgagg 1560
gatttaaaag ggaaagccaa taggggaaag atggatgtat caggagtgca ggtacctgtg 1620
ggagctatta caacaattga ggatccagtt ttagcaaaga aagtacctga gacatttcct 1680
gaattgaagc ctggagaatc cagacataca tcagatcaca tgtctattta taaattcatg 1740
ggaaggtctc atttcttgtg tacttttact tttaattcaa acaataaaga gtacacattt 1800
ccaataactc tgtcttcgac ttctaatcct cctcatggtt taccatcaac attaaggtgg 1860
ttctttaatt tgtttcagtt gtatagagga ccattggatt tgacaattat aatcacagga 1920
gccactgatg tggatggtat ggcctggttt actccagtgg gccttgctgt cgacacccct 1980
tgggtggaaa agaagtcagc tttgtctatt gattataaaa ctgcccttgg agctgttaga 2040
tttaatacaa gaagaacagg gaacattcag attagattgc catggtattc ttatttgtat 2100
gccgtgtctg gagcactgga tggcttggga gataagacag attctacatt tggattggtt 2160
tctattcaga ttgcaaatta caatcattct gatgaatatt tgtcctttag ttgttatttg 2220
tctgtcacag agcaatcaga gttctatttc cctagagctc cattaaattc aaatgctatg 2280
ttgtccactg agtccatgat gagtagaatt gcagctggag acttggagtc atcagtggat 2340
gatcccagat cagaggagga cagaagattt gagagtcata tagaatgtag gaaaccatat 2400
aaagaattga gactggaggt tgggaaacaa agaatcaaat atgctcagga agagttatca 2460
aatgaagtgc ttccacctcc taggaaaatg aaggggttat atgcttctgg aggtgaattc 2520
gat 2523

<210> 7
<211> 27
<212> ADN
<213> Chimeric Sequence

<220>
<221> primer_bind
<222> (1)..(27)
<223> Sequence # 7.
Sequence of the oligonocleotide # 7 used for
the amplification of 3A coding sequence by PCR.

<400> 7
ccatgggaat ttcagatgat gacaatg 27

<210> 8
<211> 26
<212> ADN
<213> Chimeric Sequence

<220>
<221> primer_bind
<222> (1)..(26)
<223> Sequence # 8.
Sequence of the oligonocleotide # 7 used for
the amplification of 3A coding sequence by PCR.

<400> 8
ggatatcggt tcttcctctt tgcggg 26

<210> 9
<211> 85
<212> ADN
<213> Chimeric Sequence

<220>
<221> gene
<222> (1)..(85)
<223> Sequence # 9.
      Synthetic fragment coding for 3B protein
carrying T by C and G by C nucleotide
substutions, respectively.


<400> 9
tccagctgtt ggggtttatc atggagtgac taagcccaaa caagtgatta aattggatgc 60
agatccagta gagtctcagt tgact                                       85


<210> 10
<211> 89
<212> ADN
<213> Chimeric Sequence


<220>
<221> gene
<222> (1)..(89)
<223> Sequence # 10.
      Synthetic fragment coding for 3B protein
carrying T by C and G by C nucleotide
substutions, respectively (complementary chain).


<400> 10
ctagagtcaa ctgagactct actggatctg catccaattt aatcacttgt ttgggcttag 60
tcactccatg ataaacccca acagctgga                                   89


<210> 11
<211> 25
<212> ADN
<213> Chimeric Sequence

<220>
<221> primer_bind
<222> (1)..(25)
<223> Sequence # 11.
      Sequence of the oligonocleotide # 11 used
 for the amplification of 3C coding sequence by PCR


<400> 11
tctcagtcaa ctctagaaat agcag                                       25


<210> 12
<211> 21
<212> ADN
<213> Chimeric Sequence

<220>
<221> primer_bind
<222> (1)..(21)
<223> Sequence # 12.

Sequence of the oligonocleotide # 12 used for
the amplification of 3C coding sequence by PCR

<400> 12
ataagcttga tcaattttct t 21

<210> 13
<211> 978
<212> ADN
<213> Hepatitis A virus

<220>
<221> gene
<222> Complement((1)..(978))
<223> Sequence # 13.
Sequence corresponding to the region of 3ABC
polyprotein with proteolytic activity having
the selfprocessing sites mutated.

<400> 13
gaattcctgc agcccggggg atccatggga atttcagatg atgacaatga tagtgcagta 60
gctgagtttt tccggtcttt tccatctggt gaaccatcaa attccaagtt atctagtttt 120
ttccaagctg tcactaatca caagtgggtt gctgtgggag ctgcagttgg tattcttgga 180
ttgctagtgg gaggatggtt tgtgtataag cattttcccc gcaaagagga agaaccaatt 240
ccagctgttg gggtttatca tggagtgact aagcccaaac aagtgattaa attggatgca 300
gatccagtag agtctcagtt gactctagaa atagcaggat tagttaggaa aaatttggtt 360
cagtttggag ttggtgagaa aaatggatgt gtgagatggg tcatgaatgc cttaggagtg 420
aaggatgatt ggttgttagt accttctcat gcttataaat ttgaaaagga ttatgaaatg 480
atggagtttt atttcaatag aggtggaact tactattcaa tttcagctgg taatgttgtt 540
attcaatctt tagatgtggg attccaagat gttgttctaa tgaaggttcc tacaattccc 600
aagtttagag atattactca acattttatt aagaaaggag atgtgcctag agccttgaat 660
cgcttggcaa cattagtgac aaccgttaat ggaactccta tgttaatttc tgagggacct 720
ttaaaaatgg aagaaaaagc cacttatgtt cataagaaga atgatggtac tacggttgat 780
ttgactgtag atcaggcatg gagaggaaaa ggtgaaggtc ttcctggaat gtgtggtggg 840
gccctagtgt catcaaatca gtccatacaa aatgcaattt tgggtattca tgttgctgga 900
ggaaattcaa ttcttgtggc aaagttgatt actcaagaaa tgtttcaaaa cattgataag 960
aaaattgaaa tcaagctt 978

<210> 14
<211> 3489
<212> ADN
<213> Hepatitis A virus

<220>
<221> gene
<222> Complement((1)..(3489))
<223> Sequence # 14.
Nucleotide sequence CODING for the new
modified open reading frame (ORFm) of the
Cuban M2 strain.

<400> 14
atgaatatgt ccaaacaagg aattttccag actgttggga gtggccttga ccacatcctg 60
tccttggcag atattgagga agagcaaatg attcagtccg ttgataggac tgcagtgact 120
ggagcttctt atttcacttc tgtggaccaa tcttcagttc atactgctga ggttggctca 180
caccaaattg aacctttgaa aacctctgtt gataaacctg gttctaagaa aactcagggg 240
gagaagtttt tcttgattca ttctgctgat tggctcacta cacatgctct ctttcatgaa 300

gttgcaaaat tggatgtggt gaaactgctg tacaatgagc agtttgccgt ccaaggtttg 360
ttgagatacc atacttatgc aagatttggc attgagattc aagttcagat aaatcccaca 420
ccctttcagc aaggaggact aatctgtgcc atggttcctg gtgaccaaag ttatggttca 480
atagcatcct tgactgttta tcctcatggt ctgttaaatt gcaatatcaa caatgtagtt 540
agaataaagg ttccatttat ttatactaga ggtgcttatc attttaaaga tccacagtac 600
ccagtttggg aattgacaat cagagtttgg tcagagttga atattggaac aggaacctca 660
gcttatactt cactcaatgt tttagctagg tttacagatt tggagttgca tggattaact 720
cctctttcta cacagatgat gagaaatgaa tttagagtta gtactactga aaatgttgta 780
aatttgtcaa attatgaaga tgcaagggca aaaatgtctt ttgctttgga tcaggaagat 840
tggaagtctg atccttccca aggtggtgga attaaaatta ctcatttcac tacctggaca 900
tccattccaa ccttagctgc tcagtttcca ttcaatgctt cagattcagt tgggcaacaa 960
attaaagtta taccagtgga cccatacttt ttccagatga caaacactaa tcctgatcaa 1020
aaatgtataa cagccttggc ctctatttgt cagatgttct gcttttggag gggagatctt 1080
gttttcgatt tccaggtttt tccaaccaaa tatcattcag gtaggctgtt gttttgtttt 1140
gttcctggga atgagttaat agatgttact ggaattacat taaaacaggc aactactgct 1200
ccttgtgcag tgatggacat tacaggagtg cagtcaacct tgagatttcg tgttccttgg 1260
atttctgata caccctatcg agtgaatagg tacacgaagt cagcacatca aaaaggtgag 1320
tatactgcca ttgggaagct tattgtgtat tgttataata gattgacttc tccttctaat 1380
gttgcttctc atgttagagt taatgtttat ctttcagcaa ttaatttgga atgttttgct 1440
cctctttacc atgctatgga tgttaccaca caggttggag atgattcagg aggtttctca 1500
acaacagttt ctacagagca gaatgttcct gatccccaag ttggcataac aaccatgagg 1560
gatttaaaag ggaaagccaa taggggaaag atggatgtat caggagtgca ggtacctgtg 1620
ggagctatta caacaattga ggatccagtt ttagcaaaga aagtacctga gacatttcct 1680
gaattgaagc ctggagaatc cagacataca tcagatcaca tgtctattta taaattcatg 1740
ggaaggtctc atttcttgtg tacttttact tttaattcaa acaataaaga gtacacattt 1800
ccaataactc tgtcttcgac ttctaatcct cctcatggtt taccatcaac attaaggtgg 1860
ttctttaatt tgtttcagtt gtatagagga ccattggatt tgacaattat aatcacagga 1920
gccactgatg tggatggtat ggcctggttt actccagtgg gccttgctgt cgacacccct 1980
tgggtggaaa agaagtcagc tttgtctatt gattataaaa ctgcccttgg agctgttaga 2040
tttaatacaa gaagaacagg gaacattcag attagattgc catggtattc ttatttgtat 2100
gccgtgtctg gagcactgga tggcttggga gataagacag attctacatt tggattggtt 2160
tctattcaga ttgcaaatta caatcattct gatgaatatt tgtcctttag ttgttatttg 2220
tctgtcacag agcaatcaga gttctatttc cctagagctc cattaaattc aaatgctatg 2280
ttgtccactg agtccatgat gagtagaatt gcagctggag acttggagtc atcagtggat 2340
gatcccagat cagaggagga cagaagattt gagagtcata tagaatgtag gaaaccatat 2400
aaagaattga gactggaggt tgggaaacaa agaatcaaat atgctcagga agagttatca 2460
aatgaagtgc ttccacctcc taggaaaatg aaggggttat tttcacaagc tgaattcctg 2520
cagcccgggg gatccatggg aatttcagat gatgacaatg atagtgcagt agctgagttt 2580
ttccggtctt ttccatctgg tgaaccatca aattccaagt tatctagttt tttccaagct 2640
gtcactaatc acaagtgggt tgctgtggga gctgcagttg gtattcttgg attgctagtg 2700
ggaggatggt ttgtgtataa gcatttttcc cgcaaagagg aagaaccaat tccagctgtt 2760
ggggtttatc atggagtgac taagcccaaa caagtgatta aattggatgc agatccagta 2820
gagtctcagt tgactctaga aatagcagga ttagttagga aaaatttggt tcagtttgga 2880
gttggtgaga aaaatggatg tgtgagatgg gtcatgaatg ccttaggagt gaaggatgat 2940
tggttgttag taccttctca tgcttataaa tttgaaaagg attatgaaat gatggagttt 3000
tatttcaata gaggtggaac ttactattca atttcagctg gtaatgttgt tattcaatct 3060
ttagatgtgg gattccaaga tgttgttcta atgaaggttc ctacaattcc caagtttaga 3120
gatattactc aacattttat taagaaagga gatgtgccta gagccttgaa tcgcttggca 3180
acattagtga caaccgttaa tggaactcct atgttaattt ctgagggacc tttaaaaatg 3240
gaagaaaaag ccacttatgt tcataagaag aatgatggta ctacggttga tttgactgta 3300
gatcaggcat ggagaggaaa aggtgaaggt cttcctggaa tgtgtggtgg ggccctagtg 3360
tcatcaaatc agtccataca aaatgcaatt ttgggtattc atgttgctgg aggaaattca 3420
attcttgtgg caaagttgat tactcaagaa atgtttcaaa acattgataa gaaaattgaa 3480
atcaagctt 3489

<210> 15
<211> 51
<212> ADN
<213> Chimeric Sequence

<220>

<221> gene
<222> (1)..(51)
<223> Sequence # 15.
      Synthetic fragment that reverts the
      transcription start of the vp2 protein.

<400> 15
gggatggata ttgaggaaga gcaaatgatt cagtccgttg ataggactgc a          51


<210> 16
<211> 47
<212> ADN
<213> Chimeric Sequence

<220>
<221> gene
<222> (1)..(47)
<223> Sequence # 16.
      Synthetic fragment that reverts the transcription
      start of the vp2 protein (complementary chain).


<400> 16
gtcctatcaa cggactgaat catttgctct tcctcaatat ccatccc               47


<210> 17
<211> 3426
<212> ADN
<213> Hepatitis A virus

<220>
<221> gene
<222> Complement((1)..(3426))
<223> Sequence # 17
Sequence coding for the modified open reading
frame (ΔORFm) of the Cuban M2 strain of the HAV.
This sequence does not have the gene coding
for the VP4 protein.

<400> 17
gggatggata ttgaggaaga gcaaatgatt cagtccgttg ataggactgc agtgactgga 60
gcttcttatt tcacttctgt ggaccaatct tcagttcata ctgctgaggt tggctcacac 120
caaattgaac ctttgaaaac ctctgttgat aaacctggtt ctaagaaaac tcagggggag 180
aagtttttct tgattcattc tgctgattgg ctcactacac atgctctctt tcatgaagtt 240
gcaaaattgg atgtggtgaa actgctgtac aatgagcagt ttgccgtcca aggtttgttg 300
agataccata cttatgcaag atttggcatt gagattcaag ttcagataaa tcccacaccc 360
tttcagcaag gaggactaat ctgtgccatg gttcctggtg accaaagtta tggttcaata 420
gcatccttga ctgtttatcc tcatggtctg ttaaattgca atatcaacaa tgtagttaga 480
ataaaggttc catttattta tactagaggt gcttatcatt ttaaagatcc acagtaccca 540
gtttgggaat tgacaatcag agtttggtca gagttgaata ttggaacagg aacctcagct 600
tatacttcac tcaatgtttt agctaggttt acagatttgg agttgcatgg attaactcct 660
ctttctacac agatgatgag aaatgaattt agagttagta ctactgaaaa tgttgtaaat 720
ttgtcaaatt atgaagatgc aagggcaaaa atgtcttttg ctttggatca ggaagattgg 780
aagtctgatc cttcccaagg tggtggaatt aaaattactc atttcactac ctggacatcc 840
attccaacct tagctgctca gtttccattc aatgcttcag attcagttgg gcaacaaatt 900
aaagttatac cagtggaccc atactttttc cagatgacaa acactaatcc tgatcaaaaa 960
tgtataacag ccttggcctc tatttgtcag atgttctgct tttggagggg agatcttgtt 1020
ttcgatttcc aggtttttcc aaccaaatat cattcaggta ggctgttgtt ttgttttgtt 1080
cctgggaatg agttaataga tgttactgga attacattaa aacaggcaac tactgctcct 1140
tgtgcagtga tggacattac aggagtgcag tcaaccttga gatttcgtgt tccttggatt 1200

tctgatacac cctatcgagt gaataggtac acgaagtcag cacatcaaaa aggtgagtat 1260
actgccattg ggaagcttat tgtgtattgt tataatagat tgacttctcc ttctaatgtt 1320
gcttctcatg ttagagttaa tgtttatctt tcagcaatta atttggaatg ttttgctcct 1380
ctttaccatg ctatggatgt taccacacag gttggagatg attcaggagg tttctcaaca 1440
acagtttcta cagagcagaa tgttcctgat ccccaagttg gcataacaac catgagggat 1500
ttaaaaggga aagccaatag gggaaagatg gatgtatcag gagtgcaggt acctgtggga 1560
gctattacaa caattgagga tccagtttta gcaaagaaag tacctgagac atttcctgaa 1620
ttgaagcctg gagaatccag acatacatca gatcacatgt ctatttataa attcatggga 1680
aggtctcatt tcttgtgtac ttttactttt aattcaaaca ataaagagta cacatttcca 1740
ataactctgt cttcgacttc taatcctcct catggtttac catcaacatt aaggtggttc 1800
tttaatttgt ttcagttgta tagaggacca ttggatttga caattataat cacaggagcc 1860
actgatgtgg atggtatggc ctggtttact ccagtgggcc ttgctgtcga caccccttgg 1920
gtggaaaaga agtcagcttt gtctattgat tataaaactg cccttggagc tgttagattt 1980
aatacaagaa gaacagggaa cattcagatt agattgccat ggtattctta tttgtatgcc 2040
gtgtctggag cactggatgg cttgggagat aagacagatt ctacatttgg attggtttct 2100
attcagattg caaattacaa tcattctgat gaatatttgt cctttagttg ttatttgtct 2160
gtcacagagc aatcagagtt ctatttccct agagctccat taaattcaaa tgctatgttg 2220
tccactgagt ccatgatgag tagaattgca gctggagact tggagtcatc agtggatgat 2280
cccagatcag aggaggacag aagatttgag agtcatatag aatgtaggaa accatataaa 2340
gaattgagac tggaggttgg gaaacaaaga atcaaatatg ctcaggaaga gttatcaaat 2400
gaagtgcttc cacctcctag gaaaatgaag gggttatttt cacaagctga attcctgcag 2460
cccgggggat ccatgggaat ttcagatgat gacaatgata gtgcagtagc tgagtttttc 2520
cggtcttttc catctggtga accatcaaat tccaagttat ctagtttttt ccaagctgtc 2580
actaatcaca agtgggttgc tgtgggagct gcagttggta ttcttggatt gctagtggga 2640
ggatggtttg tgtataagca tttttcccgc aaagaggaag aaccaattcc agctgttggg 2700
gtttatcatg gagtgactaa gcccaaacaa gtgattaaat tggatgcaga tccagtagag 2760
tctcagttga ctctagaaat agcaggatta gttaggaaaa atttggttca gtttggagtt 2820
ggtgagaaaa atggatgtgt gagatgggtc atgaatgcct taggagtgaa ggatgattgg 2880
ttgttagtac cttctcatgc ttataaattt gaaaaggatt atgaaatgat ggagttttat 2940
ttcaatagag gtggaactta ctattcaatt tcagctggta atgttgttat tcaatcttta 3000
gatgtgggat tccaagatgt tgttctaatg aaggttccta caattcccaa gtttagagat 3060
attactcaac attttattaa gaaaggagat gtgcctagag ccttgaatcg cttggcaaca 3120
ttagtgacaa ccgttaatgg aactcctatg ttaatttctg agggaccttt aaaaatggaa 3180
gaaaaagcca cttatgttca taagaagaat gatggtacta cggttgattt gactgtagat 3240
caggcatgga gaggaaaagg tgaaggtctt cctggaatgt gtggtggggc cctagtgtca 3300
tcaaatcagt ccatacaaaa tgcaattttg ggtattcatg ttgctggagg aaattcaatt 3360
cttgtggcaa agttgattac tcaagaaatg tttcaaaaca ttgataagaa aattgaaatc 3420
aagctt 3426

<210> 18
<211> 19
<212> ADN
<213> Chimeric Sequence

<220>
<221> sig_peptide
<222> (1)..(19)
<223> Sequence #18.
      Synthetic fragment corresponding to the
      KDEL endoplasmic reticulum retention signal
      sequence.

<400> 18
atcaaggatg aattgtaat 19

<210> 19
<211> 21
<212> ADN
<213> Chimeric Sequence

<220>
<221> sig_peptide
<222> (1)..(21)
<223> Sequence #19.
      Synthetic fragment corresponding to the KDEL
      endoplasmic reticulum retention signal sequence.

<400> 19
cgattacaat tcatccttga t                                              21


<210> 20
<211> 55
<212> ADN
<213> Chimeric Sequence

<220>
<221> D_segment
<222> (1)..(54)
<223> Sequence # 20
      synthetic fragment modifying the 3' end of
      the 2A protein and introduces a space-bar
      between this one and the KDEL signal.

<400> 20
cctaggaaaa tgaaggggtt atatgcttct ggaggtgaat tcgatatcaa ggatg      55


<210> 21
<211> 54
<212> ADN
<213> Chimeric Sequence

<220>
<221> D_segment
<222> (1)..(54)
<223>.Sequence # 21.
      synthetic fragment modifying the 3 ' end of
      the 2A protein and introduces a space-bar
      between this one and the KDEL signal.

<400> 21
aattcatcct tgatatcgaa ttcacctcca gaagcatata accccttcat tttc       54


<210> 22
<211> 2555
<212> ADN
<213> Hepatitis A virus

<220>
<221> gene
<222> Complement((1)..(2555))
<223> Sequence # 22.
      sequence coding for the structural P1-2A
      proteins joined to endoplasmic reticulum
      retention signal.

<400> 22
atgaatatgt ccaaacaagg aattttccag actgttggga gtggccttga ccacatcctg 60
tccttggcag atattgagga agagcaaatg attcagtccg ttgataggac tgcagtgact 120
ggagcttctt atttcacttc tgtggaccaa tcttcagttc atactgctga ggttggctca 180

caccaaattg aacctttgaa aacctctgtt gataaacctg gttctaagaa aactcagggg 240
gagaagtttt tcttgattca ttctgctgat tggctcacta cacatgctct ctttcatgaa 300
gttgcaaaat tggatgtggt gaaactgctg tacaatgagc agtttgccgt ccaaggtttg 360
ttgagatacc atacttatgc aagatttggc attgagattc aagttcagat aaatcccaca 420
ccctttcagc aaggaggact aatctgtgcc atggttcctg gtgaccaaag ttatggttca 480
atagcatcct tgactgttta tcctcatggt ctgttaaatt gcaatatcaa caatgtagtt 540
agaataaagg ttccatttat ttatactaga ggtgcttatc attttaaaga tccacagtac 600
ccagtttggg aattgacaat cagagtttgg tcagagttga atattggaac aggaacctca 660
gcttatactt cactcaatgt tttagctagg tttacagatt tggagttgca tggattaact 720
cctctttcta cacagatgat gagaaatgaa tttagagtta gtactactga aaatgttgta 780
aatttgtcaa attatgaaga tgcaaggcca aaaatgtctt ttgctttgga tcaggaagat 840
tggaagtctg atccttccca aggtggtgga attaaaatta ctcatttcac tacctggaca 900
tccattccaa ccttagctgc tcagtttcca ttcaatgctt cagattcagt tgggcaacaa 960
attaaagtta taccagtgga cccatacttt ttccagatga caaacactaa tcctgatcaa 1020
aaatgtataa cagccttggc ctctatttgt cagatgttct gcttttggag gggagatctt 1080
gttttcgatt tccaggtttt tccaaccaaa tatcattcag gtaggctgtt gttttgtttt 1140
gttcctggga atgagttaat agatgttact ggaattacat taaaacaggc aactactgct 1200
ccttgtgcag tgatggacat tacaggagtg cagtcaacct tgagatttcg tgttccttgg 1260
atttctgata caccctatcg agtgaatagg tacacgaagt cagcacatca aaaaggtgag 1320
tatactgcca ttgggaagct tattgtgtat tgttataata gattgacttc tccttctaat 1380
gttgcttctc atgttagagt taatgtttat ctttcagcaa ttaatttgga atgttttgct 1440
cctctttacc atgctatgga tgttaccaca caggttggag atgattcagg aggtttctca 1500
acaacagttt ctacagagca gaatgttcct gatccccaag ttggcataac aaccatgagg 1560
gatttaaaag ggaaagccaa taggggaaag atggatgtat caggagtgca ggtacctgtg 1620
ggagctatta caacaattga ggatccagtt ttagcaaaga aagtacctga gacatttcct 1680
gaattgaagc ctggagaatc cagacataca tcagatcaca tgtctattta taaattcatg 1740
ggaaggtctc atttcttgtg tacttttact tttaattcaa acaataaaga gtacacattt 1800
ccaataactc tgtcttcgac ttctaatcct cctcatggtt taccatcaac attaaggtgg 1860
ttctttaatt tgtttcagtt gtatagagga ccattggatt tgacaattat aatcacagga 1920
gccactgatg tggatggtat ggcctggttt actccagtgg gccttgctgt cgacacccct 1980
tgggtggaaa agaagtcagc tttgtctatt gattataaaa ctgcccttgg agctgttaga 2040
tttaatacaa gaagaacagg gaacattcag attagattgc catggtattc ttatttgtat 2100
gccgtgtctg gagcactgga tggcttggga gataagacag attctacatt tggattggtt 2160
tctattcaga ttgcaaatta caatcattct gatgaatatt tgtcctttag ttgttatttg 2220
tctgtcacag agcaatcaga gttctatttc cctagagctc cattaaattc aaatgctatg 2280
ttgtccactg agtccatgat gagtagaatt gcagctggag acttggagtc atcagtggat 2340
gatcccagat cagaggagga cagaagattt gagagtcata tagaatgtag gaaaccatat 2400
aaagaattga gactggaggt tgggaaacaa agaatcaaat atgctcagga agagttatca 2460
aatgaagtgc ttccacctcc taggaaaatg aaggggttat atgcttctgg aggtgaattc 2520
gatatcaagg atgaattgta atcgataccg tcgac 2555

<210> 23
<211> 1012
<212> ADN
<213> Hepatitis A virus

<220>
<221> gene
<222> Complement((1)..(1012))
<223> Sequence # 23.
Sequence coding for the 3ABC polyprotein
and for the endoplasmic reticulum
retention signal.

<400> 23
gaattcctgc agcccggggg atccatggga atttcagatg atgacaatga tagtgcagta 60
gctgagtttt tccggtcttt tccatctggt gaaccatcaa attccaagtt atctagtttt 120
ttccaagctg tcactaatca caagtgggtt gctgtgggag ctgcagttgg tattcttgga 180
ttgctagtgg gaggatggtt tgtgtataag cattttcccc gcaaagagga agaaccaatt 240
ccagctgttg gggtttatca tggagtgact aagcccaaac aagtgattaa attggatgca 300
gatccagtag agtctcagtt gactctagaa atagcaggat tagttaggaa aaatttggtt 360

cagtttggag ttggtgagaa aaatggatgt gtgagatggg tcatgaatgc cttaggagtg 420
aaggatgatt ggttgttagt accttctcat gcttataaat ttgaaaagga ttatgaaatg 480
atggagtttt atttcaatag aggtggaact tactattcaa tttcagctgg taatgttgtt 540
attcaatctt tagatgtggg attccaagat gttgttctaa tgaaggttcc tacaattccc 600
aagtttagag atattactca acattttatt aagaaaggag atgtgcctag agccttgaat 660
cgcttggcaa cattagtgac aaccgttaat ggaactccta tgttaatttc tgagggacct 720
ttaaaaatgg aagaaaaagc cacttatgtt cataagaaga atgatggtac tacggttgat 780
ttgactgtag atcaggcatg gagaggaaaa ggtgaaggtc ttcctggaat gtgtggtggg 840
gccctagtgt catcaaatca gtccatacaa aatgcaattt tgggtattca tgttgctgga 900
ggaaattcaa ttcttgtggc aaagttgatt actcaagaaa tgtttcaaaa cattgataag 960
aaaattgaaa tcaagcttcg acctcgaatc aaggatgaat tgtaatcgat ac 1012

<210> 24
<211> 2492
<212> ADN
<213> Hepatitis A virus

<220>
<221> gene
<222> Complement((1)..(2492))
<223> Sequence # 24
Sequence coding for the structural region,
whitout the vp4 protein, fused to endoplasmic
reticulum retention signal.

<400> 24
gggatggata ttgaggaaga gcaaatgatt cagtccgttg ataggactgc agtgactgga 60
gcttcttatt tcacttctgt ggaccaatct tcagttcata ctgctgaggt tggctcacac 120
caaattgaac ctttgaaaac ctctgttgat aaacctggtt ctaagaaaac tcagggggag 180
aagtttttct tgattcattc tgctgattgg ctcactacac atgctctctt tcatgaagtt 240
gcaaaattgg atgtggtgaa actgctgtac aatgagcagt ttgccgtcca aggtttgttg 300
agataccata cttatgcaag atttggcatt gagattcaag ttcagataaa tcccacaccc 360
tttcagcaag gaggactaat ctgtgccatg gttcctggtg accaaagtta tggttcaata 420
gcatccttga ctgtttatcc tcatggtctg ttaaattgca atatcaacaa tgtagttaga 480
ataaaggttc catttattta tactagaggt gcttatcatt ttaaagatcc acagtaccca 540
gtttgggaat tgacaatcag agtttggtca gagttgaata ttggaacagg aacctcagct 600
tatacttcac tcaatgtttt agctaggttt acagatttgg agttgcatgg attaactcct 660
ctttctacac agatgatgag aaatgaattt agagttagta ctactgaaaa tgttgtaaat 720
ttgtcaaatt atgaagatgc aaggcaaaaa atgtcttttg ctttggatca ggaagattgg 780
aagtctgatc cttcccaagg tggtggaatt aaaattactc atttcactac ctggacatcc 840
attccaacct tagctgctca gtttccattc aatgcttcag attcagttgg gcaacaaatt 900
aaagttatac cagtggaccc atactttttc cagatgacaa acactaatcc tgatcaaaaa 960
tgtataacag ccttggcctc tatttgtcag atgttctgct tttggagggg agatcttgtt 1020
ttcgatttcc aggtttttcc aaccaaatat cattcaggta ggctgttgtt ttgttttgtt 1080
cctgggaatg agttaataga tgttactgga attacattaa aacaggcaac tactgctcct 1140
tgtgcagtga tggacattac aggagtgcag tcaaccttga gatttcgtgt tccttggatt 1200
tctgatacac cctatcgagt gaataggtac acgaagtcag cacatcaaaa aggtgagtat 1260
actgccattg ggaagcttat tgtgtattgt tataatagat tgacttctcc ttctaatgtt 1320
gcttctcatg ttagagttaa tgtttatctt tcagcaatta atttggaatg ttttgctcct 1380
ctttaccatg ctatggatgt taccacacag gttggagatg attcaggagg tttctcaaca 1440
acagtttcta cagagcagaa tgttcctgat ccccaagttg gcataacaac catgagggat 1500
ttaaaaggga aagccaatag gggaaagatg gatgtatcag gagtgcaggt acctgtggga 1560
gctattacaa caattgagga tccagtttta gcaaagaaag tacctgagac atttcctgaa 1620
ttgaagcctg gagaatccag acatacatca gatcacatgt ctatttataa attcatggga 1680
aggtctcatt tcttgtgtac ttttactttt aattcaaaca ataaagagta cacatttcca 1740
ataactctgt cttcgacttc taatcctcct catggtttac catcaacatt aaggtggttc 1800
tttaatttgt ttcagttgta tagaggacca ttggatttga caattataat cacaggagcc 1860
actgatgtgg atggtatggc ctggtttact ccagtgggcc ttgctgtcga caccccttgg 1920
gtggaaaaga agtcagcttt gtctattgat tataaaactg cccttggagc tgttagattt 1980
aatacaagaa gaacagggaa cattcagatt agattgccat ggtattctta tttgtatgcc 2040
gtgtctggag cactggatgg cttgggagat aagacagatt ctacatttgg attggtttct 2100

attcagattg caaattacaa tcattctgat gaatatttgt cctttagttg ttatttgtct 2160
gtcacagagc aatcagagtt ctatttccct agagctccat taaattcaaa tgctatgttg 2220
tccactgagt ccatgatgag tagaattgca gctggagact tggagtcatc agtggatgat 2280
cccagatcag aggaggacag aagatttgag agtcatatag aatgtaggaa accatataaa 2340
gaattgagac tggaggttgg gaaacaaaga atcaaatatg ctcaggaaga gttatcaaat 2400
gaagtgcttc cacctcctag gaaaatgaag gggttatatg cttctggagg tgaattcgat 2460
atcaaggatg aattgtaatc gataccgtcg ac 2492

## Description

### Field of the invention.

This invention is related with the biotechnology branch and more specifically with the expression of recombinant proteins in transgenic plant cells and the use of these plants as antigen vaccines. In particular, it is shown recombinant antigens of hepatitis A virus obtained in transgenic plants derived from the expression of modified fragments of the genome of HAV from the M2 strain isolated in Cuba.

It is also demonstrated the usefulness of these antigens to develop immune response in animals after inoculation by different ways.

### Previous art.

The genome of the HAV is a simple strain RNA with possitive polarity. It has approximately 7.5 kb codified for a 253 kDa polyprotein (Cohen *et al*., Journal of Virology (1987), 61:3035-3039). The polyprotein suffers both tranlational and post-translational processes, creating structural mature proteins (VP1, VP2, VP3, VP4 and 2A) and no structural proteins (2B, 2C, 3A, 3B, 3C and 3D).

The protease 3C (Pᵣₒ3C), present in the P3 domain of the viral polypotein, is the protease which takes part in the cleavage of the polyprotein of HAV (Martín *et al*., J Virol. (1999), 73(8):6220-7), allowing the liberation of the intermediates P1-2A, 2BC and P3, which are processed afterwards. Therefore, for adequate formation of the envelop and for the replication of the HAV it is necessary the occurrence of a differential proteolitic process of the HAV polyprotein. During the processing of the P3 region, just the site 3C/3D is efficiently cleaved. There is a retarded processing in the sites 3A/3B and/or 3B/3C, allowing the accumulation of the intermediary polypeptide 3ABC (Kusov *et al*., Journal of Virology (1999), 73:9867-9878), which cleaves the polypeptide P1-2A with similar efficiency of the protease 3C. This step facilitates more efficiency in the peptamers formation. The characteristic form of the virus comes from the unification of the viral proteins and its three dimentional configuration is important for the generation of a protective immune response. The virion of the HAV shows an immunodominant antigenic site of neutralization, which is strictly preserved among strains of HAV isolated from different geographic areas. It is arranged by five conformational epitopes, three of them in the pentamers and the two others which are created after the assembling of the pentamers to form the envelope..

It is considered that these latest epitopes are formed due to conformational changes at the antigenic site or due to juxtaposition of epitope fragments present in the pentamers during their assembly. Both pentamers as well as the viral particles induce neutralization antibodies and therefore they can be useful for vaccine development (Stapleton *et al*., Journal of Virology (1993), 67:1080-1085). Using recombinant Baculoviruses containing the complete Open Reading Frame of the HAV, a giant polyprotein of the HAV was expressed. Other intermediary proteins as result of its processing in insect cells were also expressed (Stapleton *et al*., The Journal of Infectious Diseases (1995), 171:9-14). In addition, recombinants vaccinia viruses were constructed which expressed the same polyprotein of the HAV in mammalian cells. Extracts of cells infected with these genetic constructions showed a post-translational processing of the polyprotein rendering capsides similar to those of the HAV (Winokur *et al*., Journal of Virology (1994), 65:5029-5036). There are patents describing variants of recombinant vaccines against HAV expressed in baculovirus systems and vaccinias, such as: Publication of Patent Application No WO9301279 Winokur *et al*. On January 21, 1993.; patent No US5294548 (McLinden *et al*., March, 1994); Publication of Patent Application WO09844122 (Probst, August 27, 2002); Publication of Patent Application WO9111460 and patent US5605692 (Thomas *et al*. On February 25, 1997), where the sequences of the Open Reading Frame (ORF) are used for the production of imunogenic capsids and pentamers, and the methods for the obtention of the capsid of the HAV, expressing the structural regions and the region P3 in orientation cis, trans as well as bicistronics constructions are protected.

### Transgenic plant as bioreactors.

The first transgenic plants originated from gene transference by the rhizobacterium *Agrobacterium tumefaciens* were produced at the beginning of 80's decade (Zambryski et al., EMBO J. 1983, 2: 2143-2150). This technology was initially used as a way to achieve resistance to pathogenic microorganisms (Powell et al., Science 1986, 232: 738-743), to insects (Vaeck et al., Nature 1987, 328: 33-37), and to herbicides (Of Block et al., 1987, EMBO J. 6: 2513-2518). But the demonstration of the capacity of the vegetable cells to ensemble correctly foreign proteins of a high structural complexity rapidly indicated its possible value as a new strategy for escalating the economic production of recombinants proteins of industrial and biopharmaceutical interest (Barta *et al*., Plant Mol. Biol 1986, 6: 347-357; Cramer *et al*., Ann. N And Acad. Sci. 1996, 792: 62-71; Staub *et al*., Nature Biotechn. 2001. 18: 333-338).

In 1992, a new concept related to the production of subunits vaccines was introduced. It came from the demonstration that the transgenic plants could express the surface antigen of hepatitis B (HBsAg). Based on these findings it was thought that plants might be used to produce vaccine candidates in edible products and to achieve immunization only by the consumption of these products. By these facts, the denomination of **"edible vaccines"** appeared (Arntzen *et al*., Plants. Vaccine 1994, 94:339-344). Later it was demonstrated that mice fed with transgenic potatoes containing the HBsAg showed a primary immune response similar to the one obtained when a unique dose of a commercial vaccine is administered by intraperitoneal way. These results indicated that the expression of antigens in edible plants tissues can be considered as a new route of immunization (Richter *et al*., Nature Biotechnology 2000, 18:1167-1171).

There are several patents describing the use of plants for the expression of vaccines, such as: patent No US5484719 (Lam *et al*., January 16, 1996; patent No US5612487 (Lam *et al*., January 16, 1996); patent No US5914123, divisional of the patent No US5612487 and a continuation in part of the Patent Application No PCT/US94/02332 (Arntzen *et al*., June 22, 1999); patent No US6136320 (Arntzen *et al*., June 22. 1999); Publication of Patent Application WO9612801 (Arntzen *et al*., May 28, 2002) and the Patent Application No US2002006411 (Lam et al., On June 4, 2002).

The documents previously mentioned describe the use of the plants as vaccines, as well as the expression of the HBsAg in plants and in some cases they use the term "viral hepatitis" to refer to the virus of the hepatitis B (VHB). The VHB differs significantly from the virus of hepatitis A with very different characteristics and therefore they belong to different generos from the taxonomic point of view. To achieve recombinant proteins of HAV capable of raising an immunological important response it is necessary to express several proteins of the viral genome and then to achieve that such particles are formed as pentamers or empty capsids. The processing and the formation of immunogenic particles have been achieved only in eukariotic systems as vaccinias and baculoviruses, but no in simpler systems such as yeasts. In transgenic plants, complex antigens as HAV have not been expressed. In the case of the VHB, the antigen is formed by only one protein which is efficiently particulated in simple eucariotic systems such as yeast. Due to the previously exposed arguments we believe that the expression of the HBsAg does not include the expression of pentamers or empty capsides of the HAV in plants. In other patent applications is specifically described the expression of different viral antigens, such as antigens of the virus of the human papilloma in the patent applications No WO0161022 of Sohn *et al*., of August 23, 2001; that of the virus of the aftosa fever in the patent applications No CN1319670 of Zhong *et al*., of October 31, 2001; of the rotavirus in the patent applications WO0159070 of Reads *et al*., of August 16, 2001 and that of the gumboro virus in the patent applications No WO0197839 of Shachar *et al*., of December 27, 2001.

The production of recombinant proteins in plants offers many potential advantages to generate pharmaceutical compounds or vaccines of importance in clinical medicine. First, the plant systems are more economic than the industrial infrastructure used in fermentation systems or in bioreactors. Secondly, the technology to harvest and to try plants and its products to industrial scale is already available. In third position, the requirement of purification of the compound can be eliminated when the plant that contains recombinant protein is used as food (as in case of the edible vaccines). In fourth place, it is possible to direct the recombinant proteins to certain intracellular compartments as mitochondria, vacuoles, chloroplasts and endoplasmatic reticule, or to express them directly in these compartments (for example in chloroplasts). In fifth place, the risks to the health for possible pollution of the recombinant product with human beings pathogens are minimal. Finally, plants as expression systems of the recombinant proteins of pharmaceutical importance have as additional advantages the fact that many of the steps of the secretory route, including folding, assembly, glicosylation at the level of endoplasmtic reticule are similar to the cells of mammals (Ma y Hein, Plant Physiol. 1995, 109: 341- 346; Rayon *et al*., J. Exp. Bot. 1998, 49: 1463-1472; Sanderfoot y Raikhel, Plant Cell. 1999, 11: 629-641; Vitale and Denecke, Plant Cell 1999, 11: 615-628; Lerouge *et al*., Pharmaceutical Biotechnology 2000, 1: 347-354).

### Detailed description of the invention.

Basic design of the object of this invention is supported by genetic constructions that allow the combined expression of genes that codify for different variants of structural proteins and mutated non-structural regions, directed to the recombinant expression of antigenic pentamers and capsids of HAV in transgenic plants capable of generating an immune response.

The novelty of our invention fundamentally falls in the regions of the viral genome used for the conformation of a new Open Reading Frame coding for a polyprotein of minor size, shaped by strictly structural regions (only up to the protein 2A) and the viral modified protease which presents a superior size to the protease 3C of the virus due to the fact that the sites of cleavage between the proteins 3A/3B and 3B/3C are mutated. The expression in the cytosol and in the endoplasmatic reticulum of viral capsids of the HAV in transgenic plants is achieved by the first time, specially under the control of the promoters and regulatory signals used. The pentamer and capsids formation in endoplasmatic reticulum as a product of the combined expression of the structural region and the region responsible for the proteolysis, demonstrates the possibilities of this compartment for the assembly and the storage of complex structures, such as the case of the HAV. The production of pentamers and capsids in plants allows us the possibility to employ them as bioreactors to obtain a cheap and sure vaccine.

The invention is demonstrated by means of examples in which transgenic tobacco, rice and carrot plants were used for the obtaining by the first time of inmunogenic capsids and pentamers of the virus in vegetable cells. The capsids and pentamers of HAV, resultant of the invention, can be used as antigens vaccines and also in diagnostic assays for HAV detection.

### Genetic constructions.

### Obtaining cDNA of the HAV.

From the RNA of the strain M2 of the HAV isolated in Cuba, the nucleotide sequence coding for the Open Reading Frame (ORF) of the virus was amplified, using Reverse Transcription Technology - Polymerase Chain Reaction (RT-PCR). This fragment was cloned in a plasmid and its nucleotide sequence was determined. It presents differences that generate variations in 11 aminoacid residues with respect to the reported sequences. The analysis of the sequences allows to classify the strain M2 as part of the subgenotype IA, to which belong almost all of the American strains. From the genome of this strain, modified fragments were designed and constructed and then were used in different genetic constructions that are object of this invention,

### Genetic constructions of vectors for the expression of capsids and pentamers in transgenic plants.

### Recombinant protease of HAV.

To allow the formation of viral capsids it is necessary that a differentially proteolitic processing of the polyprotein happens which allows the ordered releasing of viral proteins. The efficiency of capsid formation increases when the intermediary 3ABC is present due to the hydrophobic interaction of the protein 3AB with the membrane and the viral proteins. The cleavage sites of the protease 3C between the proteins 3A/3B and 3A/3C were mutated to obtain a polypeptide 3ABC from which the protease 3C is not released and in turn preserves its proteolytic function, necessary for formation of the pentamers and capsids of the HAV,. Substitutions were glutamic by valine between 3A/3B and serine by leucine between 3B/3C. This polypeptide was used in the design of novel and different strategies for the expression of HAV proteins forming immunogenic capsids and pentamers.

### Recombinant HAV for the expression of capsids and pentamers in the cytosol of the vegetable cell.

In HAV, the polypeptide P1-2A has an important function in the formation of the viral capsid. In this polypeptide there are two signs that regulate the formation of the capsid. In its carboxil terminal domain is found the protein 2A, which is needed in the first stages of capsid assembling to achieve the formation of pentamers. They are formed from the combination of five not processed molecules of the polypeptide P1-2A. The protein VP4 is needed in the second stage for the association of the pentamers and the formation of the capsids.

For the expression of the modified polyprotein in the citosol of the vegetable cell, there were constructed vectors that contain the sequence of modified Open Reading Frame (ORFm). These constructs codifies for a significantly minor size polyprotein (compared to the original polyprotein of the HAV). This sequence is the result of the fusion of P1-2A polypeptide coding sequence and the sequence that codifies for the mutated protease 3ABC.

The plasmid vector used for the transformation of plants, by means of *A. tumefaciens*, contains DNA sequences that codifies for proteins of the HAV fused to the nucleotides sequences coding for regulatory signals of their expression in plants. In this case, the sequence coding for the protein is not fused to any specific signal of transport across the secretory route of the vegetable cell, so it is expressed in the citosol of the cell.

### Recombinant HAV for exclusive expression of pentamers in the citosol of the vegetable cell.

Autoprocess and form exclusively immunogenic viral pentamers. The minor size of the pentamers regarding the capsids allows to achieve higher levels of expression because it provokes a minor As it has been previously described, the protein VP4 as part of the polypeptide VP0 is required for the association of the pentamers and the formation of the viral capsids.

From the nucleotide sequence coding for the polyprotein ORFm, the fragment that codes for the protein VP4 was eliminated, giving place to a sequence called ΔORFm. A plasmid vector for the expression of the polyprotein ΔORFm fused to the sequences that regulate its expression in the citosol of the vegetable cells was constructed. It was used to obtain transgenic plants by means of the infection of leaves of tobacco, rice and carrot with *A. tumefaciens*. The polyprotein expressed from this genetic construction has a significantly minor size and it is capable to metabolic charge in the vegetable cell. The obtained product is equally feasible to be used like immunogen for the development of vaccines.

### Recombinant HAV for the expression of capsids and pentamers in the endoplasmtic reticulum of the vegetable cell.

The accumulation of heterologous proteins in the endoplasmatic reticulum in plants is achieved by means of the use of sequences to drive them to the secretory pathway, and therefore the endoplasmatic reticulum, and also of signals of retention in this organelle.

As signal peptide, a sequence that codes for the N-terminal peptide of the sweet potato sporamin was used. As signal of retention of proteins in the endoplasmatic reticulum, the sequence that codes for the peptide KDEL located in the carboxyl terminal of the protein, was used.

Sweet potato sporamin signal peptide was fused to the 5' region of P1-2A nucleotide coding sequence and a sequence for spacer peptide fused to KDEL coding sequence was inserted in the 3' region. Resulting DNA fragment was placed under regulation of plant expression signals at a binary vector. This vector included the mutated polypeptide 3ABC coding sequence fused at its 5' extreme to sweet potato signal peptide and at its 3' extreme the KDEL coding sequence. All this elements also under regulation of plant expression signals. Both polypeptides are located in the endoplasmic reticulum and the protease 3ABC is able to process the polypeptide P1-2A and achieve efficient particle formation.

### Recombinant HAV to exclusively expres pentamers in the endoplasmatic reticulum of the vegetable cell.

The polypeptide ΔP1-2A was obtained by removing the VP4 nucleotide coding sequence from the P1-2A polypeptide coding sequence. This sequence was fused at its 5' end to sweet potato sporamin signal peptide and at its 3 end', to the sequence that codifies for a spacer peptide joined to KDEL coding sequence. In the same binary vector the sequence that codes for the mutated polypeptide 3ABC was equally fused at its 5' end to the sweet potato sporamin signal peptide and at its 3' end to the KDEL coding sequence. All the elements under the signs of regulation for the expression in plants.

The two polypeptides are located in the endoplasmatic reticulum and the protease 3ABC is capable of process the polypeptide ΔP1-2A and to achieve the expression of pentamers exclusively. These plants exhibit higher levels of expression as well as a better growth and development, which bears to obtain more biomass.

### Identification of transgenic plants that express the product of the genes of the modified HAV.

*A. tumefaciens* was transformed with each of the binary vectors and there were obtained bacterial colonies containing these plasmids. *A. tumefaciens* independently carrying the different genetic constructions were used for plant transformation and finally to obtain plants resistant to the kanamicin as a selection marker. The integration of the foreign DNA in plants was verified by the use of Southern blot and PCR techniques.

From leaves of transgenic plants, extraction of soluble proteins was performed, grinding them with liquid nitrogen in a particular protein extraction buffer. Capsids and pentamers were identified using specific antiserum to the HAV and a neutralizing monoclonal antibody. Immunological methods such as Western blot, ELISA or Immunomicroscopy were performed, they demonstrated that transgenic plants express the polyprotein or in some cases the expected polypeptides, and also that they are processed and assembled in pentamers or capsids.

Plants expressing higher levels of recombinant proteins were used for capsid and pentamer purification using a neutralizing monoclonal antibody.

### Determination of the immunogenicity of the purified capsids and pentamers.

Capsid and pentamers immunopotency of HAV was determined by the immunological response of immunized mice with the purified product from plant leaves of tobacco and rice. Also the potency was evaluated in mice fed with transgenic carrots expressing the HAV antigen. Oral route and parenteral way were used as methods for the introduction of the antigen. The immune response was controlled and verified using Elisa's technology to determine the reactivity of the antiserum of the animal to the HAV, and for the neutralizing capacity of the immune sera to neutralize the HAV infection in vitro.

### Advantages of the invention.

Among the most important advantages that our invention offers are: antigenic similarity among envelopes and purified pentamers as products of the expression of our constructs and the original virus; the expression levels of envelopes and pentamers in plants as products of the constructs we claim are higher either than the ones obtained when the HAV open reading frame is expressed or when the region P1-2A and the region P3 are co-expressed, because the polyprotein obtained as product of the expression of our constructs has a significantly lower size than the original virus size; the polypeptide 3ABC is exclusively composed by the proteins 3A, 3B and 3C and has mutated the selfprocessing sites between the proteins 3A/3B and 3B/3C, it avoids the polypeptide processing and therefore the proteolitic function of the polyprotein is assumed by the polypeptide itself with higher efficiency; pentamers and HAV viral envelopes expression levels in endoplasmic reticulum of the vegetable cell are higher than in the cytoplasm; the exclusive expression of pentamers in the vegetable cell is more efficient and allows a better plant growth and development due to the lower size of these particles; the scale-up and production of pharmaceutical protein from plants is suitable to produce high amounts of antigens; production costs are reduced compared to other systems currently used and described in the state of the art; HAV antigen expression in plants decreases the contamination risks with pathogens affecting human beings; oral immunization against HAV significantly contributes to cheap the immunization costs due to the possibility to use plants without the need to purify the product.

### Microorganism Deposit.

Plasmids pBVHARE, pBΔVHARE, pBMLAm and pBΔMLAm were leave to deposit under the regulations of Budapest Treatment for Microorganism Deposit at the Belgian Coordinated collection of Microorganisms BCCM, LMBP-COLLECTION with the access numbers LMBP 4721, LMBP 4722, LMBP 4723 and LMBP 4724 respectively deposited on May 19, 2003.

### DESCRIPTION OF FIGURES.

Figure 1. Genetic constructs for envelopes and pentamers expression in plant vegetable cytosol. A) ORF scheme of the M2 strain of HAV. B) Scheme of the sequences that code for the structural proteins (P1-2A). C) Scheme of the sequences that code for the 3ABC region. D) Scheme of the ORFm of HAV. E) Scheme of the insert of interest cloned in the binary vector for plant expression.
Figure 2. Genetic constructs for pentamer expression in the cytosol of the vegetable cell. A) Scheme of the ΔORFm without the sequence that codes for VP4. B) Scheme of the insert of interest cloned in the binary vector for ΔORFm plant expression.
Figure 3. Genetic constructs for the expression of envelopes and pentamers in the endoplasmic reticulum of the vegetable cell. A) Scheme of the P1-2A sequence fused to KDEL sequence. B) Scheme of the insert of interest cloned in the binary vector for the expression in the endoplasmic reticulum. E- Spacer, K- KDEL
Figure 4. Genetic constructs for the expression of pentamers in the endoplasmic reticulum of the vegetable cell. A) Scheme of the P1-2A sequence without the VP4 sequence fused to KDEL sequence. B) Scheme of the insert of interest cloned in the binary vector for plant expression. E- Spacer, K- KDEL
Figure 5. Southern blot of genomic DNA from transgenic plants.
Figure 6. Southern blot of PCR products from carrot and rice transgenic plants.
Figure 7. Western blot of plant proteins from transgenic tobacco, carrot and rice transformed with genetic constructs for the expression in the cytosol of envelopes and pentamers.
Figure 8. Immune-enzymatic assay (ELISA) performed to tobacco, carrot and rice plants transformed with the genetic constructs for the expression in the cytosol of HAV envelopes and pentamers.
Figure 9. Electronic immune-microscopy of one tobacco plant, transformed with the construct pBMLAm. A) Untransformed plant. B) Transformed plant. C) Transformed plant.
Figure 10. Inhibition ELISA of the sera from mice immunized with HAV by intraperitoneal way.
Figure 11. Inhibition ELISA of the sera from mice orally immunized with HAV pentamers purified from rice and tobacco plants.
Figure 12. Inhibition ELISA of the sera from mice orally immunized by feeding them with carrots collected from plants expressing HAV pentamers.

### EXAMPLES.

### Example 1. Cloning of the ORF of the HAV of the Cuban strain M2.

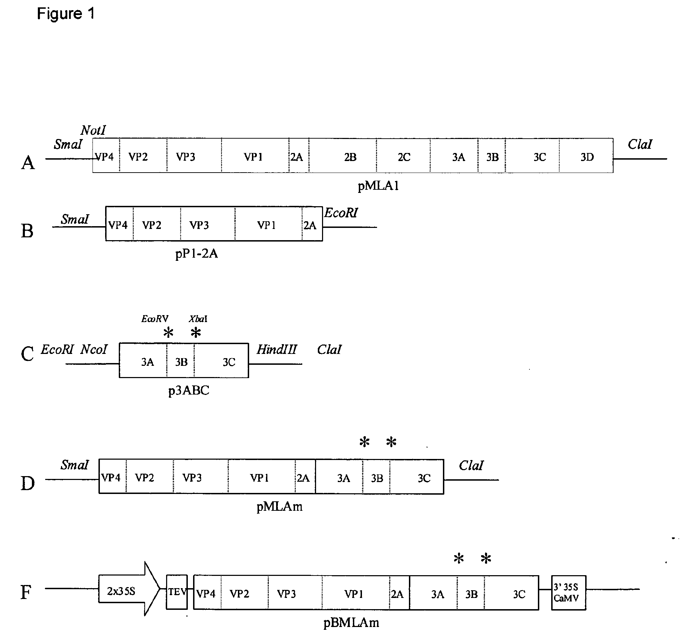

The sequence of interest of the plasmid pMLA1 is shown in the Figure 1 (A). From the HAV strain M2 isolated and characterized in Cuba, the RNA was purified and a DNA fragment of 6.7 kb was amplified by means of Reverse Transcription-Polymerase Chain Reaction technology (RT - PCR), using specific oligonucleotides (SEQ ID NO 1 and 2) for other previously reported sequences of the HAV. The band was cloned in a vector BlueScript (KS+), previously digested with *Sma*I endonuclease. The resultant palsmid was named pMLA1 and was used for the sequencing determination of HAV's ORF of the Cuban M2 strain. DNA sequence (SEQ ID NO 3) presents changes with regard to the reported ones. It has changes that generate 11 different aminoacids. The analysis of these sequences allows to classify the strain M2 inside the subgenotype IA, to which belong almost the totality of the American strains. The sequence confirms that the Cuban strain M2 is a really HAV strain different from the ones previously reported.

### Example 2. Genetic constructs for the expression of capsides and pentamers in the plant cell cytosol.

The sequence of interest of the plasmid pP1-2A is shown in the Figure 1 (B). This plasmid was obtained by PCR amplification of the sequences coding for the structural proteins (P1-2A), using specific oligonucleotides (SEQ ID NO 4) complementary for the 5' region of the sequence that codes for the protein VP4 and the 3' region of the sequence that codes for the protein 2A from the plasmid pMLA1, respectively. The amplified band of 2.5 kb (SEQ ID NO 6) was cloned in the vector BlueScript (KS+) *Sma*I digested.

To obtain the plasmid p3ABC, which sequence of interest is shown in the Figure 1 (C), the region of 0.2 kb that codes for the protein 3A was amplified by PCR, using oligonucleotides (SEQ ID NO 7 and 8) complementary for the 5' and 3' regions of this gene. It was cloned in a vector Blue Script (KS+) *BamH*I/*EcoR*V digested. After that, in the same vector but in the region among *EcoR*V/*Xba*I sites, a synthetic nucleotide sequence (SEQ ID NO 9 and 10) coding for the protein 3B was cloned. The obtained plasmid was called p3AB. On the other hand, from the plasmid pMLA1, the sequence of 0.65 kb that codes for the protein 3C was amplified by PCR using the oligonucleotides SEQ ID NO 11 and 12. This band was cloned among the sites *Xba*I/*Hind*III of the vector P3AB. The resulting sequence obtained was called 3ABC (SEQ ID NO 13),

It codes for a polyprotein with proteolitic activity but without self-processing possibilities because the cutting sites between the proteins 3A/B and 3B/C are mutated by means of the nucleotide substitution T by C and G by C respectively.

The sequence of interest of the plasmid pMLAm is shown in the Figure 1 (D). To obtain it, the plasmid pP1-2A was *EcoR*I and *Cla*I digested. A 1kb band coding for the mutated polypeptide 3ABC was extracted by *EcoR*I/*Cla*I digestion and cloned in the proper sites of the pP1-2A previously digested. The plasmid pMLAm contains the modified sequence that codes for a HAV polyprotein of a significantly minor weight compared to the original polyprotein (SEQ ID NO 14).

The plasmid pKMLAm was obtained by cloning the 3.4 kb ORFm band *Sma*I/*Cla*I digested in the vector pKTPL-2. This vector contains as promoter 2X the sequence of 35SCaMV promoter, the leader sequence of the TEV and the terminator of the 35S CaMV. To clone the ORFm band, the plasmid pKTPL-2 was *Nco*I digested followed by blunting with *Klenow* fragment of DNA *Pol*I and finally *Cla*I digested.

The sequence of interest of the binary plasmid pBMLAm is shown in the Figure 1 (F). This plasmid was obtained by *Sph*I digestion of the plasmid pKMLAm and subsequent treatment with *Mung Bean* nuclease, rendering a band of 4.7 kb that was cloned in the binary vector pBin19 previously *Sma*I digested.

The resultant plasmid pBMLAm contains: the neomycin phosphotranferase II gene (NPTII) which acts as selection marker conferring kanamycin resistance; the ORFm gene which codes for the modified HAV polyprotein, regulated by the 2X 35S CaMV promoter and the leader of the TEV as well as the terminator of the CaMV. It also has the borders sequences of T-DNA to facilitate its transference to the plant genome.

### Example 3. Genetic constructs for the expression of pentámers in the cytosol of the plant cell.

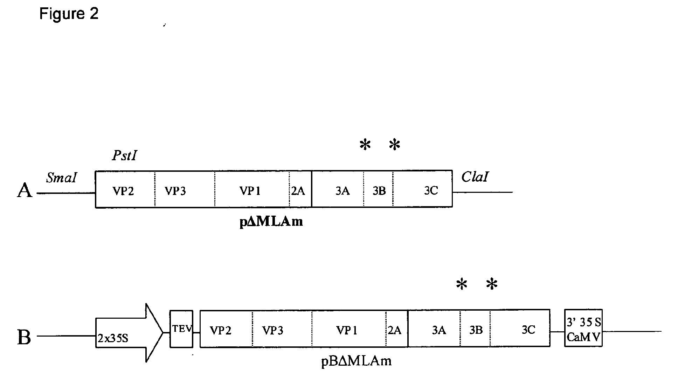

The sequence of interest of the plasmid pΔMLAm is shown in the Figure 2 (A). To eliminate the protein VP4 and to obtain this plasmid, the fragment of 114bp was eliminated from the plasmid pMLAm cutting with the enzymes *Sma*I/*Pst*I and it was replaced with the synthetic nucleotide sequence (SEQ ID NO 15 and 16) that returns the beginning of the gene that codes for the protein VP2. The sequence of the region ΔORFm corresponds to the SEQ ID NO 17. The plasmid pKΔMLAm was obtained cloning the band ΔORFm (3.46 kb) *Sma*I/*Cla*I digested in the plasmid pKTPL-2 previously digested *Nco*I/*Klenow*/*Cla*I.

The sequence of interest of the binary plasmid pBΔMLAm is shown in the Figure 2 (B), the binary plasmid was obtained by digestion of Bin19 with the enzyme *Sma*I and a DNA fragment of 4.6 kb resultant of the digestion of the plasmid pKMLAm with the enzyme *Sph*I and treatment with *Mung Bean* nuclease was then cloned.

The resultant plasmid pBΔMLAm contains: the neomycin phosphotransferase II (NPT II) gene which functions as selectable marker conferring kanamycine resistance; the ORFm gene which codes for the HAV modified polyprotein, regulated by the sequence 2X 35S CaMV as promoter and TEV leader, as well as the terminator of 35S CaMV. It also contains the right and left borders to be transferred to the plant genome.

### Example 4. Genetic constructs for the expression of capsides and pentamers in the endoplasmic reticulum of the plant cell.

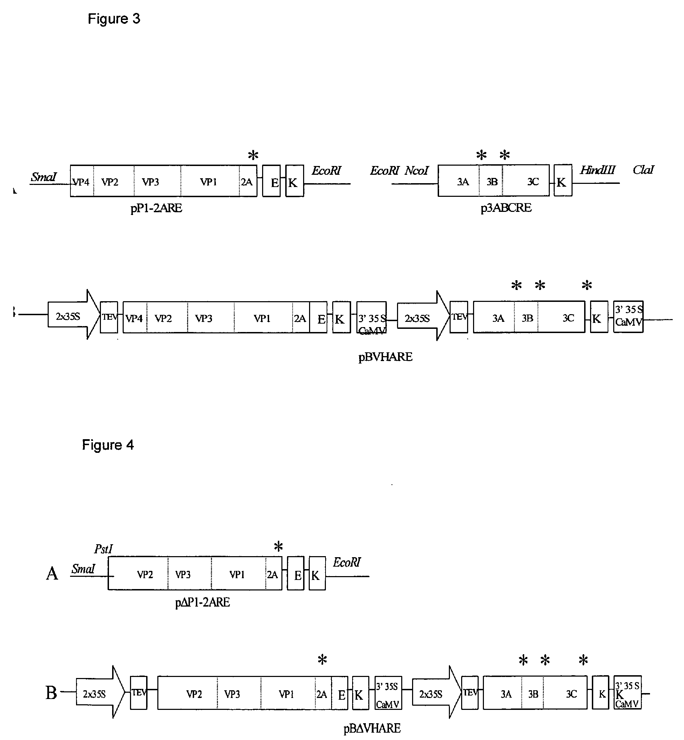

The sequence of interest of the plasmid pBVHARE appears in the Figure 3B. To obtain this plasmid, a synthetic fragment (SEQ ID NO 18 and 19) that codes for KDEL retention signal was cloned in the sites *EcoR*V/*Cla*I of the vector BS (+). On the other hand, in the sites *Sty*I/*EcoR*I of the plasmid pP1-2A, a synthetic fragment (SEQ ID NO 20 and 21) that modifies the 3' end of the protein 2A and eliminates the protease cutting site in this region, as well as introduces a sequence that works as space-bar between the union of the gene and the sequence that codes for the KDEL signal, was cloned. Later this sequence (2.5 kb) was extracted with the enzymes *Sma*I/*EcoR*V and was cloned in the vector BS-KDEL, resulting the plasmid pP1-2ARE (Figure 3A, SEQ ID NO 22). The plasmid p3ABCRE was obtained digesting the p3ABC plasmid with the enzymes *Xho*I/*Klenow*/*EcoR*I and cloning the 3ABC sequences in the *EcoR*I/*EcoR*V sites (Figure 3A, SEQ ID NO 23).

To provide plant expression regulatory signals to the genes of interest, the structural region P1-2A-KDEL (2.5 kb), extracted from the plasmid pP1-2ARE with the enzymes *Sma*I/*Cla*I, was cloned in the plasmid pKTPL-2 digested *Nco*I/*Klenow*/*Cla*I. The resulting plasmid was named pKPl-2ARE. The 1kb region 3ABC-KDEL was extracted from the plasmid p3ABCRE with the enzymes *Nco*I/*Cla*I and was cloned in the plasmid pKTPL-2 digested with the same enzymes. The resulting plasmid was pK3ABCRE.

Finally, to achieve the plasmid for plant transformation by means of *A. tumefaciens,* the 2 kb sequence extracted from the plasmid pK3ABCRE with enzyme *Sal*I was cloned in the binary vector pBin 19 previously digested with the same enzyme. Resulting plasmid was called pB3ABCRE. Later, in the *Sph*I site of the same vector, the expression cassette corresponding to the sequence P1-2A-KDEL, extracted from the plasmid pKP1-2ARE by *Sph*I digestion, was cloned. The resultant plasmid pBVHARE carries separated both the structural regions and the region with protease function, fused to the retention signals in the reticulum under plant expression regulatory signals and the neomycin phosphotransferase II (NPT II) gene as selectable marker.

### Example 5. Genetic constructs for the expression of pentamers in the endoplasmic reticulum of the plant cell.

The sequence of interest of the plasmid pBΔVHARE is shown in the Figure 4(B). This plasmid was obtained cutting the plasmid pP1-2ARE with the enzymes *Sma*I/*Pst*I and it was replaced with the synthetic nucleotide sequence (SEQ ID NO 15 and 16) that returns the beginning of the gene that codes for the protein VP2. The resultant plasmid pΔP1-2ARE (Figure 4A, SEQ ID NO 24), was digested *Sma*I/*Cla*I and the band of the 2.4 kb was cloned in the vector pKTPL-2 digested *Nco*I/*Klenow*/*Cla*I, resulting the plasmid pKΔP1-2ARE. It was cloned in the plasmid pB3ABCRE (the binary plasmid conting the 3ABC-KDEL), the expression cassette digested with the *Sph*I enzyme).

The resultant binary plasmid contains: the structural region, without the sequences that code for the protein VP4, fused to the KDEL peptide, under plant expression signals; the 3ABC-KDEL region under the same signals and the neomycin phosphotransferase II (NPT II) gene as the selectable marker.

### Example 6. Obtention of capsids and pentamers of HAV in Nicotina tabacum transgenic plants.

The genetic transformation of *Nicotiana tabacum* plants was carried out following the method of Zambryski *et al*., (1983).

The *A.tumefaciens* strain At 2260 (Deblaere *et al*., 1985) was transformed by the method of the liquid nitrogen (Hofgen and Willmitzer, 1988) with the developed binary plasmids (PBΔVHARE , PBVHARE, PBΔMLAm, PBMLAm). Leave discs of tobacco plants of the variety Petit Havana MR 1 cultivated "in vitro" were transformed with the recombinant *Agrobacteriums.* Kanamycine (100 mg/L) was used as selection marker.

Several procedures such as Southern blot, Western blot, ELISA and immunomicroscopy were performed to detect the presence of the genes of interest in the tobacco plant genome and their expression, as well as formation of either viral envelop or pentamers.

### Example 7. Obtention of capsides and pentamers of HAV in transgenic carrot plants (Daucus carota L.)

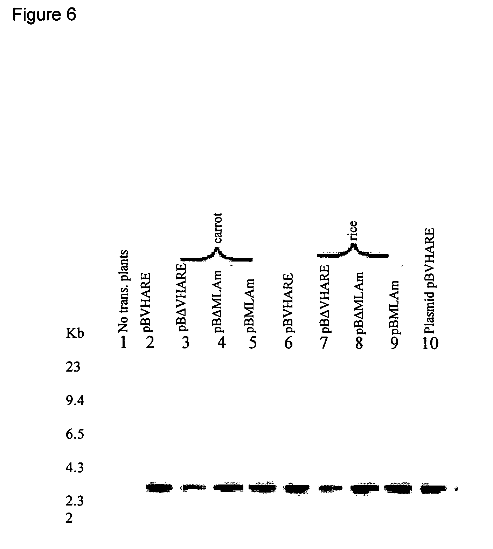

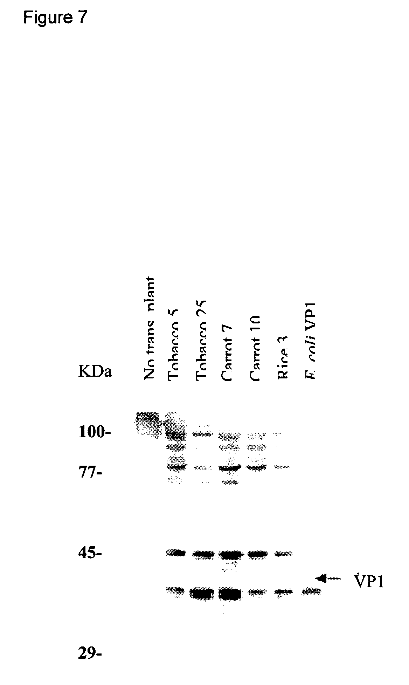

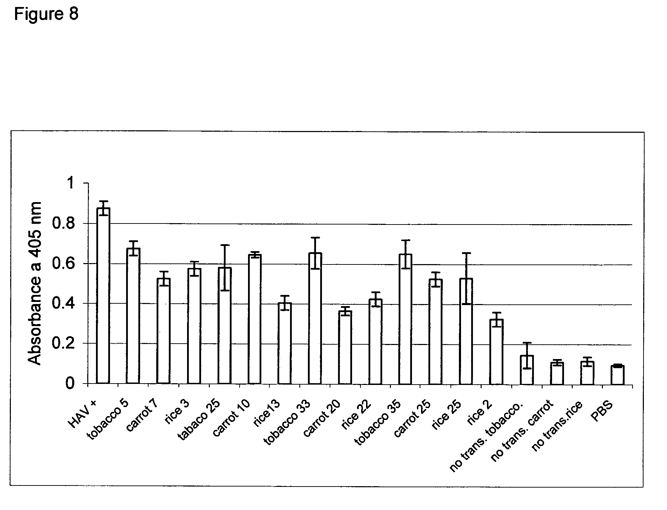

For the transformation of this plant were used The *A. tumefaciens* strain At 2260 transformed with the binary plasmids (PBΔVHARE , PBVHARE, PBΔMLAm, PBMLAm) was used for plant transformation. Germinated three weeks old hypocotyls of the variety NEW KURODA were cut in 1 cm segments and planted in BAN-9 medium (Murashige and Skoog, 1962 (MS), supplemented with 0.5 mg/L of NAA) for three days. Later they were incubated during 30 minutes with an At suspension containing each one of the constructions previously described. Again, explants were transferred to BAN-9 medium for 72 hours. After this period, they were planted in regeneration medium supplemented with Kanamycine (100 mg/l). Shoots appeared after 3 weeks and they were individualized and planted in MS medium also supplemented 100 mg/L of Kanamycine. Gene integration was verify by Southern blot of PCR products (Figure 6). Polyprotein expression, its processing and formation of viral capsids and pentamers was demonstrated by ELISA (Figure 8) and Western blot (Figure 7).

### Example 8. Obtaining of the capsid and pentamers of the HAV in transgenic rice plants (Oryza sativa L.).

The genetic transformation of rice plants was carried out following the method used by Hiei *et al*., (1994). The *A.tumefaciens* strain At2260 was transformed with the developed binary plasmids (pBΔVHARE, pBVHARE, pBΔMLAm, pBMLAm) using method of the liquid nitrogen. Callus obtained from rice escutelo were transformed with recombinant A. *tumefaciens*. Kanamycine (100 mg/L) was used as selection marker.

To verify the presence of the genes of interest in plant rice genome and their expression, as well as the formation of viral capsids or pentamers, different procedures were performed, which are below described.

### Example 9. Molecular characterization and immunochemical of the transgenic plants.

### Analysis by Southern blot.

The method described by Dellaporta *et al*., (1983) was used to obtain chromosomal DNA for Southern blot purposes from tobacco, carrot and rice plants. As samples, leaves of transformed plants with the previously described constructs that showed resistance to the selection marker were used. Leaves of untransformed plants were used as negative control.

Total DNA digestions, agarose gel electrophoresis, transfer to a Hybond N membrane and hybridization were performed by standard procedures (Sambrook *et al*., 1989). A 1.2 kb DNA fragment that includes the gene coding for VP1 protein VP1 was 32_{P} labeled by means of a Primer-a-Gene Labeling System (Promega Corp., the USA) and used as radioactive probe. The same fragment was used as positive control.

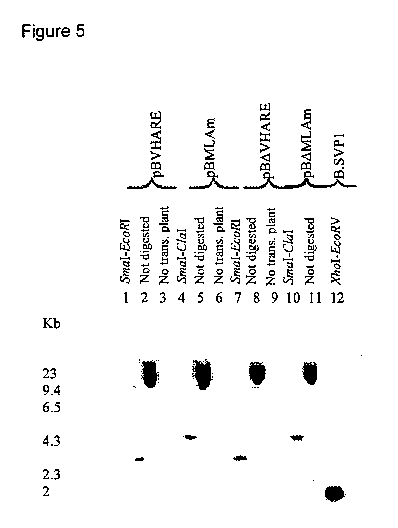

Figure 5 shows the Southern blot of transgenic tobacco plants transformed with the constructs PBΔMLAm and PBMLAm to expressing in the cytoplasm both capsids and pentamers of the HAV. By DNA digestion with *Sma*I and *Cla*I there is a resulting band of 3.4 kb. Total DNA from transgenic plants transformed with the constructs PBΔVHARE and PBVHARE to be expressed in the endoplasmic reticulum was digested with *Sma*I I-*EcoR*I resulting a band of 2.4 kb. Results shown in the Figure 5 demonstrate that plants contain in their genomes the sequences coding for the structural proteins.

Both in transgenic carrot and rice plants, a Southern blot was performed to the PCR amplification products with oligonucleotides corresponding to the sequences SEQ ID NO 4 and 5. As it is shown in the Figure 6, the radioactively labeled sequence that codes for the protein VP1 complements with a band of 2.5 kb corresponding to the expected size of the sequence that codes for the structural proteins.

### Analysis by Western blot.

Western blot results are shown in the Figure 7. Western blot assay for immunodetection of the recombinant molecules was carried out according to the methodology described by Towbin *et al*., (1979). Western blot samples consisted on total soluble proteins extracted from transgenic tobacco, carrot and rice plants, transformed with the constructs to express only pentamers: Clones tobacco 5, carrot 7 and rice 3, transformed with the construct pBΔVHARE to express pentamers in the endoplasmic reticulum; and clones tobacco 25 and carrot 10, transformed with the construct pBΔMLAm which allows the expression of the pentamers in the cytoplasm. As negative controls, protein extracted from untransformed tobacco leaves were used. As positive control, the VP1 protein expressed in *E. coli.* Leaves were grinded with liquid nitrogen up to obtain a very thin dust. 1 mL of protein extraction buffer [Tris-HCl 61 mM pH 6.8, Triton 0.1 %, glycerol 12.5% and Fluoride of Fenilmetilsulfonilo (PMSF) 1 mM] per gram of leaves was added as reported Schouten *et al*., (1997). The insoluble material was removed by centrifugation at 13 000 rpm.

Total proteins of SDS-PAGE were transferred to a nitrocellulose membrane and proteins of interest were identified using a polyclonal antibody anti-VP1 conjugated to the enzyme alkaline phosphatase (PhoA). The enzyme detection was performed by means of a colorimetrical reaction.

In the Figure 7 is possible to observe the presence of a protein band of the same size of the VP1 protein in all cultures, as well as other intermediate products resulting from incomplete processing of the polyprotein.

### Immunoenzymatic test (ELISA).

ELISA results are shown in the Figure 8. "Sandwich" assays were performed. The plate (Maxisorp, Nunc) was covered with 10 mg/mL of the monoclonal antibody 7E7 in carbonate buffer (Na2CO3 0.015 M, NaHCO3 0.028 M, pH 9.6) during 4 hours at 37 °C. Blocking was carried out during 2 hours at 37 °C, with 5% of milk in PBS (NaCl 100 mM, Na₂PO₄ 80 mM, NaH₂PO₄ 20 mM, pH 7.4). Later 100 µL of the samples corresponding to transformed and untransformed tobacco, carrot and rice plants (prepared in the same way as was described for the Western blot), were added. Plate was incubated overnight at 4°C. After washing with PBS, 100 µL of the monoclonal antibody 7E7, conjugated with alkaline phosphatase at 1/1000 dilution (1 mg/mL in PBS containing 0.5% milk) were added. The plate was incubated at 37 °C during 1 hour. The reaction was developed by addition of 4-nitrofenilfosfatoe (substrate of the enzyme), prepared in 0.1% of Diethanolamine. Color appearance was followed during a 60 minutes period. The absorbance was read at a wavelength of 405nm in an spectrophotometer. The washes of the plate in every stage of the ELISA were performed three times with PBS containing 0.1% of Tween 20.

### Analysis for electronic immunomicroscopy.

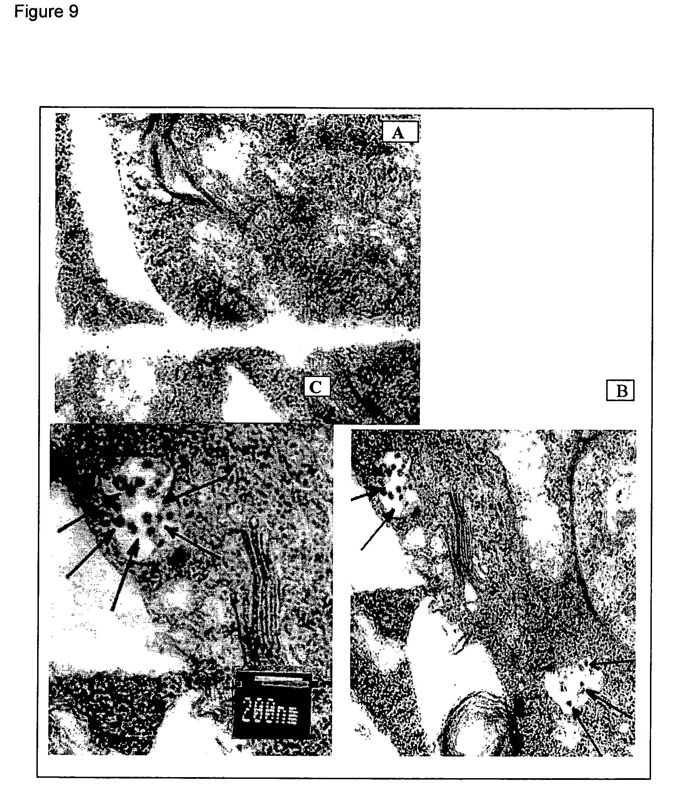

Immunomicroscopy results are shown in the Figure 9. Samples of transformed tobacco with the plasmid pBMLAm and untransformed plants, both from tissue culture, were fixed in a solution of 4% of Formaldehyde and later in 0.2% of Glutaraldehyde. They were dehydrated in ethanol and then incubated in a Lowicryl K 4M (Chemische Werke Lowi, Waldkraiburg) solution. Ultra thin cuts were placed in a nickel grill and incubated with the monoclonal antibody 7E7. After this step, there was an incubation period with the polyclonal antibody anti-mouse IgG labeled with 15 nm of gold colloidal particles (British Bio-Cell International). Immunolabeled sections were contrasted by a 5 minutes treatment in Uranilacetate followed by 7 minutes in Lead Citrate, before being examined by a transmission electronic microscope (Jeol-Jem 2000EX, Japan). Results show particles of approximately 27 nm of diameter only in the tobacco transformed plants with the construction pBMLAm, by means of what the protein was expressed in the cell cytoplasm.

### Example 10. Purification of capsids and pentamers from transgenic tobacco and rice plants.

For capsid and pentamer purification a monoclonal antibody anti HAV obtained at CIGB laboratories that exclusively recognizes the particles and immunogenic pentamers was used.

Vegetable cell proteins were extracted using the protocol described for Western blot analysis. Supernatant resultant from centrifugation was dissolved in 0.5M of sodium chloride and mixed with an affinity gel (Bio-rad Laboratories, Richmond, CA) charged with an antibody. The mix was incubated for 16 hours at 4°C. The gel was washed with 10 volumes of PBS (NaCl 100 mM, Na₂PO₄ 80 mM, NaH₂PO₄ 20 mM, pH 7.4) and later the protein of interest was eluted with glycine 0.2 M at pH 2.5. The eluate was neutralized with basic Tris and was dialyzed against PBS. The presence of HAV particles and pentamers from these leave extracts was detected by ELISA, using a 7E7 commercial monoclonal antibody (Mediagnost), specific for recognition of HAV viral capsids and pentamers.

### Example 11. Determination of the inmunogenicity of the capsids and pentamers purified from transgenic plants by means of the intraperitonial administration.

White ICR mice of 14 weeks old were immunized with two doses of 750 EL.U of capsids and pentamers purified from transgenic tobacco and rice plants. In the same way, a group of mice were inoculated with a commercial HAV antigen (Mediagnost) and used as positive control. Another group was inoculated with PBS and used as negative control. Blood samples were collected 0, 15, 30, 50 and 70 days post-inoculation.

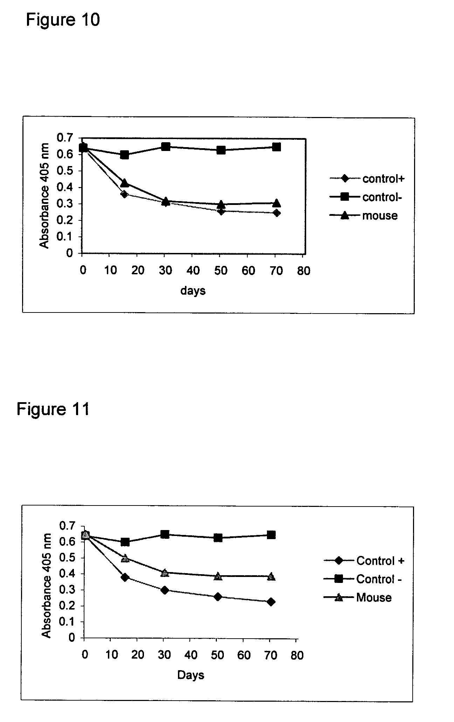

The antibody levels were measured by means of an inhibition ELISA: the plate was covered with 5 µg of the monoclonal antobody 7E7 and then incubated for 4 hours. Later it was washed once with PBS-0.1% Tween. Blocking was performed adding 5 % of milk in PBS-0.1% Tween and incubated for 2 hours at 37 °C. The plate was washed 3 times with PBS-0.1% Tween. Sera from immunized mice, previously incubated during 20 min at 37°C, with HAV antigen (Mediagnoct), were added. The plate was incubated for 12 hours at 16°C and washed 5 times with PBS-0.1% Tween. Finally, 100µL of the monoclonal antibody 7E7 conjugated with alkaline phosphatase diluted 1/1000 in PBS containing 0.5% milk, were added. The incubation was carried out during an hour at 37°C. The reaction was developed by addition of 4-nitrophenilphosphate (enzyme substrate) prepared in diethanolamyne. Color appearance was followed during 60 minutes. The absorbance was read to a wavelength of 405 nm in an spectrophotometer. In the Figure 10 is shown the average levels of inhibition of the sera of the mice inoculated with the antigen purified from transgenic plants, detected in blood of the mice immunized with pentamers produced by tobacco and rice plants. In the same way, similar levels of antibody were observed in mice immunized with the antigen produced in tobacco and rice plants transformed with the constructs that allow the expression of capsids and pentamers.

### Example 12. Determination of the immunogenicity of the capsids and pentamers purified from transgenic plants by means of the oral administration.

The antigen oral administration was performed by two routes: using the purified antigen and feeding animals with carrots expressing the antigen.

For antigenicity determination of purified capsids and pentamers administered by oral way, pentamers and capsids were administered to 8 weeks Balb/c mice in four doses of 7500

EL.U. 200 µL of blood at 0, 15, 30, 50 and 70 days post - inoculation were collected to detect the presence of anti HAV antibodies by an ELISA of inhibition.

ELISA of inhibition was performed by means of the procedure described previously in the example 11.

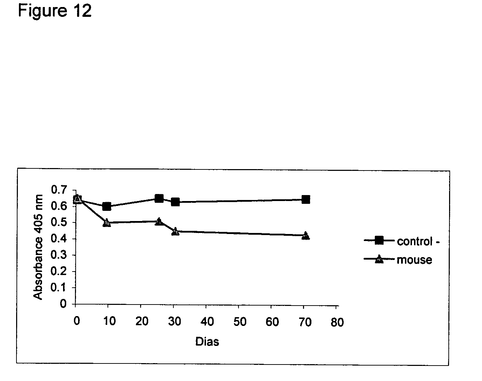

According to the results shown in the Figure 11, the oral administration of the HAV pentamers expressed in transgenic plants produces immune response that is demonstrated by average inhibition of the sera from mice used in the experiment. Average inhibition of the sera from mice orally administered were lower compared with the ones obtained after intraperitoneal administration. Pentamers oral administration through plants was carried out with 5 g of row carrots (transformed with the construct pBΔMLAm especifically designed to produce only pentamers) once a week during 4 weeks., sera from mice fed with untransformed carrots, were used as negative control. Ability of these plants to rise an immune response was demonstrated by an inhibition ELISA shown in the Figure 12.

## Claims

1. Hepatitis A virus recombinant antigens wherein they are obtained in vegetable cells transformed with genetic constructs which contain chimeric HAV genes based on modified fragments of the HAV genome (SEQ ID NO 3).

2. Hepatitis A virus recombinant antigens according to the claim 1 **characterized by** they contain only pentamers.

3. Hepatitis A virus recombinant antigens according to the claims 1 and 2 **characterized by** they are obtained from the expression of a chimeric gene according to SEQ ID NO 17 that contains the fusion of the following elements:
a. A nucleotide sequence that codes for the proteins VP2, VP3, VP1 and 2A (SEQ ID NO 25)
b. A nucleotide sequence that codes for the proteins 3A, 3B, 3C. (SEQ ID NO 13)

4. Hepatitis A virus recombinant antigens according to the claim 3 wherein the chimeric gene is expressed in vegetable cells regulated by the appropriate promoter and terminator signals.

5. Hepatitis A virus recombinant antigens according to the claim 4 **characterized by** they are obtained in the cytoplasm of the vegetable cell.

6. Hepatitis A virus recombinant antigens according to the claim 5 **characterized by** they are expressed in dicotyledonous plants.

7. Hepatitis A virus recombinant antigens according to the claim 6 **characterized by** they are expressed in tobacco, carrot and fruits of edible plants.

8. Hepatitis A virus recombinant antigens according to the claim 5 **characterized by** they are expressed in monocotyledonous plants.

9. Hepatitis A virus recombinant antigens according to the claim 8 **characterized by** they are expressed in rice and in fruits of edible plants.

10. Hepatitis A virus recombinant antigens according to the claim 1 **characterized by** they contain pentamers and empty capsids.

11. Hepatitis A virus recombinant antigens according to the claim 10 **characterized by** they were obtained from the expression of a chimeric gene that contains the fusion of the two following elements:
a. A nucleotide sequence according to the SEQ ID NO 6 coding for the proteins VP4, VP2, VP3, VP1 and 2A.
b. A nucleotide sequence coding for the proteins 3A, 3B and 3C according to the claim 3b.

12. Hepatitis A virus recombinant antigens according to the claim 11 wherein the chimeric gene is expressed in the vegetable cell regulated by appropriate promoter and terminator signals.

13. Hepatitis A virus recombinant antigens according to the claim 12 **characterized by** they are obtained in the cytoplasm of the vegetable cell.

14. Hepatitis A virus recombinant antigens according to the claim 13 **characterized by** they are obtained in dicotyledonous plants.

15. Hepatitis A virus recombinant antigens according to the claim 14 **characterized by** they are expressed in tobacco, carrot and fruits of edible plants.

16. Hepatitis A virus recombinant antigens according to the claim 13 **characterized by** they are obtained in monocotyledonous plants.

17. Hepatitis A virus recombinant antigens according to the claim 16 **characterized by** they are expressed in rice and fruits of edible plants.

18. Hepatitis A virus recombinant antigens according to the claim 2 wherein they are obtained from the coordinated expression of the two chimeric genes:
a. A nucleotide sequence, according to the sequence ID No.24 coding for the proteins VP2, VP3, VP1, 2A, fused at its 5' end to a signal sequence and at its 3' end to an spacer sequence followed by the sequence coding for the KDEL peptide.
b. A nucleotide sequence according to the sequence ID No. 23 coding for the proteins 3A, 3B, 3C referred in the claim 3B, fused at its 5'end to a signal sequence and at its 3' end to an spacer sequence followed by the sequence coding for the KDEL peptide.

19. Hepatitis A virus recombinant antigens as in the claim 18 **characterized by** chimeric genes are expressed in the vegetable cell regulated by appropriate promoter and terminator signals.

20. Hepatitis A virus recombinant antigens as in the claims 18 and 19 wherein they are obtained in the endoplasmic reticulum of the vegetable cell.

21. Hepatitis A virus recombinant antigens as in the claim 20 **characterized by** they are obtained in dycot plants.

22. Hepatitis A virus recombinant antigens as in the claim 21 **characterized by** they are obtained in tobacco, carrot and fruits of edible plants.

23. Hepatitis A virus recombinant antigens as in the claim 20 **characterized by** they are obtained in monocot plants.

24. Hepatitis A virus recombinant antigens as in the claim 23 **characterized by** they are obtained in rice and fruits of edible plants.

25. Hepatitis A virus recombinant antigens as in the claim 10 wherein they are obtained from the coordinated expression of two chimeric genes.
a. A nucleotide sequence according to the sequence ID No. 22 coding for the proteins VP4, VP2, VP3, VP1, 2A fused at its 5'end to a signal sequence and at its 3' end to an spacer sequence followed by the sequence coding for the KDEL peptide.
b. A nucleotide sequence according to the claim 18b.

26. Hepatitis A virus recombinant antigens as in the claim 25 **characterized by** chimeric genes are expressed in the vegetable cell regulated by appropriate promoter and terminator signals.

27. Hepatitis A virus recombinant antigens as in the claims 25 and 26 **characterized by** they are obtained in the endoplasmic reticulum of the vegetable cell.

28. Hepatitis A virus recombinant antigens as in the claim 27 **characterized by** they are obtained in dycot plants.

29. Hepatitis A virus recombinant antigens as in the claim 28 **characterized by** they are obtained in tobacco, carrot and fruits of edible plants.

30. Hepatitis A virus recombinant antigens as in the claim 27 **characterized by** they are obtained in monocot plants.

31. Hepatitis A virus recombinant antigens as in the claim 30 **characterized by** they are obtained in rice and fruits of edible plants.

32. Hepatitis A virus recombinant antigens as in the claims 1, 3, 11, 18 and 25 which can be purified to be administered by parenteral way.

33. Hepatitis A virus recombinant antigens as in the claim 32 which can be administered in combination with other viral antigens.

34. Hepatitis A virus recombinant antigens as in the claims 1, 3, 11, 18 and 25 which can be administered by oral way.

35. Hepatitis A virus recombinant antigens as in the claim 34 which can be administered as lyophilized extract, pill or capsule.

36. Hepatitis A virus recombinant antigens as in the claims 1, 3, 11, 18 and 25 which can be administered in juice form.

37. Hepatitis A virus recombinant antigens as in the claims 1, 3, 11, 18 and 25 which are immunogenic and rise protective immune response against Hepatitis A.

38. Hepatitis A virus recombinant antigens as in the claim 32 which can be used as part of a diagnostic kit for Hepatitis A.

39. The use of the antigens referred in the claims 1 to 38 to prepare simple and combined vaccines.
